(19) Europäisches Patentamt
European Patent Office
Office européen des brevets

(11) Veröffentlichungsnummer: **0 243 429**
**B1**

(12) **EUROPÄISCHE PATENTSCHRIFT**

(45) Veröffentlichungstag der Patentschrift: **23.01.91**

(51) Int. Cl.[5]: **C 07 D 277/64**, C 07 F 9/547, C 07 H 17/00, C 07 H 19/20, C 07 K 5/00, C 07 K 7/00, G 01 N 33/535

(21) Anmeldenummer: **86906321.4**

(22) Anmeldetag: **24.10.86**

(86) Internationale Anmeldenummer: **PCT/EP86/00615**

(87) Internationale Veröffentlichungsnummer: **WO 87/02667 07.05.87 Gazette 87/10**

# (54) D-LUCIFERIN-DERIVATE UND DEREN VERWENDUNG.

(30) Priorität: **24.10.85 DE 3537877**

(43) Veröffentlichungstag der Anmeldung: **04.11.87 Patentblatt 87/45**

(45) Bekanntmachung des Hinweises auf die Patenterteilung: **23.01.91 Patentblatt 91/04**

(84) Benannte Vertragsstaaten: **AT BE CH DE FR GB IT LI LU NL SE**

(56) Entgegenhaltungen:
**EP-A-0 024 525**
**EP-A-0 087 959**

**Journal of Organic Chemistry, Vol. 30, No. 7, July 1965, Washington, D.C. (US) E.H. White et al.: "Analogs of firefly luciferin", see pages 2344, 2345, 2347**

**Biochemistry, Vol. 9, No. 5, March 1970 Washington, D.C. (US) M.J. Cormier et al.: "Studies on the bioluminescence of Renilla reniformis. VII. Conversion of luciferin into luciferyl sulfate by luciferin sulfokinase", pages 1184-1189, see pages 1184, 1186, 1187, 1188**

(73) Patentinhaber: **Geiger, Reinhard, Dr.**
**Eisenhartstrasse 6**
**D-8000 München 60 (DE)**
(73) Patentinhaber: **Miska, Werner, Dr.**
**Haylerstrasse 8**
**D-8000 München 50 (DE)**

(72) Erfinder: **Geiger, Reinhard, Dr.**
**Eisenhartstrasse 6**
**D-8000 München 60 (DE)**
Erfinder: **Miska, Werner, Dr.**
**Haylerstrasse 8**
**D-8000 München 50 (DE)**

(74) Vertreter: **Köster, Hajo, Dr.**
**Jaeger, Steffens & Köster Patentanwälte**
**Pippinplatz 4a**
**D-8035 München-Gauting (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 243 429 B1

(56) Entgegenhaltungen:
Chemical Abstracts, Vol. 58, No. 4, 18 February 1963, Columbus, Ohio (US) H.H. Seliger et al.: "Chemiluminescence of firefly luciferin without enzyme", see column 3648a

Journal of the American Chemical Society, Vol. 102, No. 9, 23 April 1980, Washington D.C. (US) E.H. White et al.: "Chemi- and bioluminescence of firefly luciferin" see pages 3199, 3208, in particular pages 3204, 3205

**Beschreibung**

Die Erfindung betrifft D-Luciferin-Derivate, deren Verwendung und Verfahren zur Detektion von mit einem Enzym markierten Liganden bei der Bestimmung von Substanzen, insbesondere biochemisch aktiver Substanzen.

Die Bestimmung von Substanzen, die bei biologischen Prozessen eine Rolle spielen, die biologisch wirksam sind, die bei biologischen Prozessen auftreten etc. stellt ein großes Problem dar.

So ist beispielsweise auf dem immunologischen Gebiet die Bestimmung von Antigenen, Haptenen oder Antikörpern äußerst wichtig.

Die Entwicklung der ersten Radio-Immunoassays, die zur Bestimmung von derartigen Antigenen oder Antikörpern eingesetzt werden können, liegt mehr als 20 Jahre zurück. Seitdem sind verschiedene immunologische Techniken entwickelt worden, bei denen markierte Reaktanten (Tracer) zur Bestimmung von Antigenen oder Antikörpern eingesetzt werden.

Von besonderer Bedeutung sind die Enzym-Immunoassays, bei denen Enzyme als Marker dienen. Derartige Enzym-Immunoassays setzen sich im Vergleich zu den Radio-Immunoassays immer mehr durch, da bei den Enzym-Immunoassays kein radioaktiver Abfall anfällt, da die Reagentien länger aufbewahr werden können, und da der Tracer nicht zerstört wird.

Bei den Enzym-Immunoassays unterscheidet man zwischen den homogenen (homogeneous enzyme immuno assays; EMIT) und den heterogenen (heterogeneous enzyme immuno assays; ELISA).

Eine Übersicht über die wichtigsten Techniken derartiger Enzym-Immunoassays findet sich in:

J. Clin. Chem. Clin. Biochem., Bd. 18, 1980, Seiten 197—208, "Enzyme immuno assays in clinical chemistry: present status and trends" von M. Oellerich und

Principles of Enzyme Immuno Assays von M. Oellerich in Methods of Enzymatic Analysis, Bd. 1, H. U. Bergmeyer, ed., S. 233—260, Verlag Chemie, Weinheim/Bergstraße (1983).

Einen zusammenfassenden Artikel über die Verwendungsmöglichkeiten von Enzymen in Immunoassay-Techniken findet sich in:

Analyst, Mai 1984, Vol. 109, Seiten 533—547, "Use of Enzymes in Immuno Assay Technics, a Review" by Christopher Blake and Barry J. Gould.

Bei derartigen Immunoassays dienen die Enzyme als Marker. Dabei koppelt man die Enzyme mit einem Liganden zu einem Ligand-Enzymkonjugat. Bei diesem Liganden kann es sich um einen Antikörper, ein Antigen oder ein Hapten handelt. Einen mit einem Enzym markierten Liganden (beispielsweise ein mit einem Enzym-markierten Antikörper) setzt man dann mit seinem biochemischen "Gegenstück" (beispielsweise einem Antigen) um, wobei sich der Ligand an sein biochemisches Gegenstück anlagert.

Zur Detektion der Ligand-Enzymkonjugate setzt man diese mit einem Substrat um. Die Enzyme setzen aus dem Substrat ein Reaktionsprodukt frei, das man analytisch bestimmen kann, beispielsweise fotometrisch, potentionmetrisch, thermometrisch oder mittels Szintillationsspektrometrie.

Bei diesen Enzym-Immunoassays nutzt man somit den Verstärkungseffekt über den Substratumsatz der Enzyme pro Zeitenheit aus. Man kann dadurch die Menge des gebildeten Reationsproduktes und somit auch die Menge an gebundenen, mit einem Enzym-markierten Liganden (Antikörper, Antigen, Hapten) quantitativ bestimmen.

Die Qualität eines Enzym-Immunoassays hängt somit unter anderem von dem Verstärkungseffekt und der Detektionsgrenze für das Reaktionsprodukt ab.

Häufig sind jedoch derartige Enzym-Immunoassays nicht empfindlich genug. Dies kann dadurch bedingt sein, daß der Verstärkungseffekt bei dem Substratumsatz nicht groß genug ist oder daß die Detektionsgrenze für das Reaktionsprodukt zu hoch liegt.

Es wurde nun überaschend gefunden, daß man Luciferin-Derivate als Substrate zur Detektion von mit Enzymen markierten Liganden einsetzen kann. Aus den Luciferin-Derivaten wird durch die Enzyme die Verbindung Luciferin freigesetzt, die wiederum mit einem Enzym, nämlich der Luciferase des Leuchtkäfers Photinus pyralis, unter Abgabe von Licht umgesetzt werden kann. Aus der dabei abgestrahlten Lichtmenge kann man die Menge an Ligand-Enzymkonjugat quantitativ bestimmen, woraus sich wiederum die Menge an zu bestimmender Substanz errechnen läßt.

Aufgabe der vorliegenden Erfindung ist es daher, ein verbessertes Detektionssystem für mit Enzymen markierte Liganden und dabei einsetzbare Verbindungen bereitzustellen.

Diese Aufgabe wird gelöst durch die Schaffung von D-Luciferin-Derivaten der folgenden allgemeinen Formel (I):

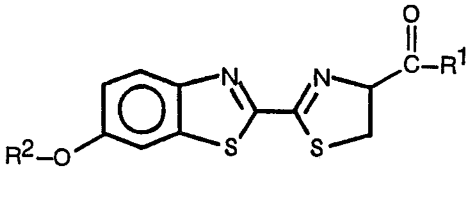

(I)

worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurest, der über die α-Aminogruppe gebunden ist, oder einen

Oligopeptidrest mit bis zu 10 L-Aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_{20}$-Alkyl- oder $C_2$—$C_{20}$-Alkenylgruppe, die gegebenenfalls durch einen oder mehrere Phenylrest(e) substituiert ist, eine Arylgruppe mit 6 bis 18 C-Atomen, eine Gruppe der allgemeinen Formel (II):

$$R^3-\overset{\overset{\displaystyle O}{\|}}{C}- \qquad (II)$$

worin $R^3$ eine gerade oder verzweigte $C_1$—$C_{20}$-Alkyl- oder $C_2$—$C_{20}$-Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist, oder ein glykosidisch angeknüpftes Mono- oder Disaccharid bedeutet, mit der Ausnahme von D-Luciferin, O-Methylluciferin und D-Luciferin-methylester.

Der D-Luciferin-methylester wurde von E. H. White in J. Amer. Chem. Soc. 102, 3199 (1980) beschrieben. Das O-Methylluciferin (Verbindung der allgemeinen Formel (I) mit $R^1$ = OH und $R^2$ = $CH_3$) ist bekannt aus J. Org. Chem., Bd. 30, Nr. 7, (1965).

Bei den Alkyl- oder Alkoxygruppen der Reste $R^1$, $R^2$ und $R^3$ handelt es sich insbesondere um solche mit 1 bis 8, vorzugsweise 1 bis 6 und insbesondere bevorzugt 1 bis 4, Kohlenstoffatomen.

Die Alkenyloxy- oder Alkenylgruppen der Substituenten $R^1$, $R^2$ und $R^3$ weisen insbesondere 2 bis 8, vorzugsweise 2 bis 6 und insbesondere bevorzugt 2 bis 4 Kohlenstoffatome auf.

Als L-Aminosäurereste setzt man vorzugsweise solche von natürlich vorkommenden Aminosäuren ein. Auch das Oligopeptid ist vorzugsweise aus diesen natürlich vorkommenden Aminosäuren aufgebaut.

Bei den Arylgruppen handelt es sich beispielsweise um die Phenyl- oder Naphthylgruppe. Bei dem Monosaccharid handelt es sich beispielsweise um einen Galactose-, Glucose-, Manose- oder Fucoserest.

Als Nucleotide kann man folgende einsetzen: Adenosin-, Guanosin-, Thymidin-, Cytidin- oder Uridinmono-, -di-, oder -tri-phosphat.

Bevorzugte Luciferin-Derivate sind solche der allgemeinen Formel (I), worin

$R^1$ eine Hydroxy- oder Aminogruppe, eine $C_{1-6}$-Alkoxy- oder $C_2$—$C_6$-Alkenyloxy, eine natürlich vorkommende Aminosäure oder ein Oligopeptid aus natürlich vorkommenden Aminosäuren mit vorzugsweise bis zu 5 Aminosäureeinheiten bedeutet und

$R^2$ ein Wasserstoffatom, eine $H_2PO_3$- oder $HSO_3$-Gruppe, eine gerade oder verzweigte $C_1$—$C_{20}$-Alkyl- oder $C_2$—$C_{20}$-Alkenylgruppe, die gegebenenfalls durch einen oder mehrere Phenylrest(e) substitutiert ist, eine Arylgruppe mit 6 bis 18 C-Atomen, eine Gruppe der allgemeinen Formel (II):

$$R^3-\overset{\overset{\displaystyle O}{\|}}{C}- \qquad (II)$$

worin $R^3$ eine gerade oder verzweigte $C_1$—$C_{20}$-Alkyl- oder $C_2$—$C_{20}$Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist.

Weiterhin bevorzugte Luciferin-Derivate der allegemeinen Formel (I) sind solche, worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurerest, der über die α-Aminogruppe gebunden ist, oder einen Oligopeptidrest mit bis zu 10 L-Aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_6$-Alkyl- oder $C_2$—$C_6$-Alkenylgruppe, die gegebenenfalls durch einen Phenylrest substituiert ist oder eine Gruppe der allegemeinen Formel (II)

$$R^3-\overset{\overset{\displaystyle O}{\|}}{C}- \qquad (II)$$

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

Weiterhin bevorzugte Luciferin-Derivate sind solche der allgemeinen Formel (I), worin

$R^1$ eine Hydroxy- oder Aminogruppe, eine $C_{1-6}$-Alkoxy- oder $C_2$—$C_6$-Alkenylgruppe, ein Aminosäurerest einer natürlich vorkommenden Aminosäure oder ein Oligopeptidrest aus natürlich vorkommenden Aminosäuren mit vorzugsweise bis zu 5 Aminosäure- einheiten bedeutet und

$R^2$ ein Wasserstoffatom, ein $H_2PO_3$- oder $HSO_3$-Gruppe, eine gerade oder verzweigte $C_1$—$C_6$-Alkyl- oder

$C_2$—$C_6$-Alkenylgruppe, die gegenebenfalls durch einen Phenylrest substituiert ist oder eine gruppe der allgemeinen Formel (II)

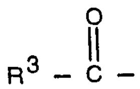

(II)

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

Weiterhin bevorzugte Luciferin-Derivate sind solche der allgemeinen Formel (I), worin

$R^1$ eine Hydroxy- oder Aminogruppe, eine $C_{1-6}$-Alkoxygruppe oder eine Gruppe der allgemeinen Formel (III)

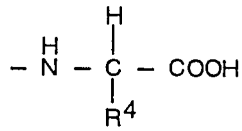

(III)

bedeutet, worin $R^4$ für

-H, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2\ C_6H_5$, 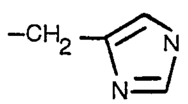 , 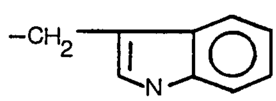 , $-CH_2SH$,

$-CH_2CH_2CH_2-NH-C(=NH)-NH_2$ oder $CH_2CH_2SCH_3$,

steht, oder worin die Gruppe der allgemeinen Formel (III) ein Prolin- oder Hydroxyprolinrest ist, und

$R^2$ ein Wasserstoffatom, eine $C_1$—$C_6$-Alkylgruppe, die gegebenenfalls durch einen Phenylrest substituiert ist, eine Phenyl- oder Naphthylgruppe, eine $H_2PO_3$- oder $HSO_3$-Gruppe oder eine Gruppe der Formel II

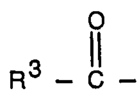

(II)

bedeutet, wobei $R^3$ eine gegebenenfalls durch einen Phenylrest substituierte $C_1$—$C_6$-Alkylgruppe darstellt.

Weiterhin bevorzugte Luciferin-Derivate sind solche der allgemeinen Formel (I), worin

$R^2$ ein Wasserstoffatom bedeutet, falls $R^1$ eine anderen Rest als eine Hydroxygruppe darstellt, oder worin $R^1$ eine Hydroxygruppe bedeutet, falls $R^2$ einen anderen Rest als ein Wasserstoffatom darstellt.

Die erfindungsgemäßen Verbindungen der allgemeinen Formel (I) sowie das O-Methylluciferin und der Luciferin-methylester können als Substrate zur Detektion von Liganden eingesetzt werden, an die ein Enzym gekoppelt ist. Das Enzym muß in der Lage sein, aus den genannten Luciferin-Derivaten die Verbindung Luciferin freizusetzen. Das freigesetzte Luciferin ist noch in geringster Konzentration mit dem Enzym Luciferase des Leuchtkäfers Photinus pyralis nachweisbar.

Auf diese Weise können somit alle Liganden detektiert werden, an die ein entsprechendes Enzym gekoppelt werden kann.

Die Funktion eines Liganden ist es, sich an eine zu diesem Liganden korrespondierende biochemische Substanz ("biochemisches Gegenstück") anzulagern (bzw. damit einen Komplex zu bilden). Nach dieser Umsetzung wird die Kombination aus markiertenm Ligand und biochemischen Gegenstück mit einem Luciferin-Derivat (Substrat) umgesetzt, wie dies nachstehend näher beschrieben ist. Aus dem Substratumsatz kann man die Menge der "Kombination" errechnen, woraus sich wiederum die Menge der zu bestimmenden Substanz ermitteln läßt.

Bei der zu bestimmenden Substanz kann es sich beispielsweise um das genannte biochemische Gegenstück handeln. Es kann sich auch um den Liganden selbst handeln, wenn man einen markierten Liganden mit einem nicht markierten Liganden unbekannter Konzentration um das genannte biochemische Gegenstück konkurrieren läßt.

Es ist auch möglich, daß weder das genannte biochemische Gegenstück noch der Ligand, sondern eine das biochemische Gegenstück anlagerbare Substanz die zu bestimmende Substanz darstellt. Dabei ist die zu bestimmende Substanz über ein Zwischenglied (z.B. probe, sonde, second antibody), welche das genannte biochemische Gegenstück darstellt, an den Liganden gebunden.

Die Luciferin-Derivate stellen somit eine neue Substrat-Klasse dar, die anstelle der bisher bekannten Substrate in Tests eingesetzt werden, bei denen mit einem Enzym markierte Liganden Anwendung finden.

Gegenstand der Erfindung ist daher auch ein Verfahren zur Detektion von Liganden bei der Bestimmung von biochemisch aktiven Substanzen, wobei man den Liganden mit einem Enzym unter Bildung eines Ligand-Enzymkonjugats markiert. Dieses Verfahren ist dadurch gekennzeichnet, daß man ein Enzym einsetzt, das aus den erfindungsgemäßen Luciferin-Derivaten der allgemeinen Formel (I), aus O-Methylluciferin oder aus dem Luciferinmethylester Luciferin freisetzen kann, mit Hilfe des Enzym-Konjugats Luciferin freisetzt, das freigesetzte Luciferin mit dem Enzym Luciferase des Leuchtkäfers Photinos pyralis umsetzt, die dabei abgestrahlte Lichtmenge quantitativ bestimmt, insbesondere luminometrisch, und anhand der erhaltenen Daten die Menge der zu bestimmenden Substanz ermittelt.

Das erfindungsgemäße Verfahren ist insbesondere bei Immunoassays anwendbar. Die Durchführung dieser Immunoassays geschieht auf bekannte Weise unter Einsatz von Antikörpern, Haptenen oder Antigenen, an die ein Enzym gekoppelt ist. Zur Detektion geht man dann wie folgt vor. Man setzt dann mit Hilfe des Enzym-Konjugats aus einem Luciferin-Derivat Luciferin frei, setzt das freigesetzte Luciferin mit dem oben spezifizierten Enzym Luciferase um, bestimmt die dabei abgestrahlte Menge quantitativ, insbesondere luminometrisch, und ermittelt anhand der erhaltenen Daten die Menge des zu bestimmenden Antigens, Antikörpers bzw. Haptens.

Bei dem erfindungsgemäßen Immunoassay konjugiert man ein Enzym auf an sich bekannte Weise mit einem Liganden (Antikörper, Antigen oder Hapten). Bei diesem Liganden kann es sich dabei um die Art von biochemisch aktiver Substanz handeln, die man zu bestimmen wünscht. Dieser Fall sei beispeilsweise an einem kompetitiven Immunoassay erläutert.

Einen derartigen kompetitiven Immunoassay kann man beispielsweise zur Bestimmung von Antigenen einsetzen. Dazu koppelt man eine bekannte Menge des Antigens mit dem Market-Enzym. Dieses mit einem Einzym-markierte Antigen, dessen Menge bekannt ist, läßt man zusammen mit einer Probe, die dasselbe Antigen, das jedoch nicht markiert ist und dessen unbekannte Menge man zu bestimmen wünscht, mit einer begrenzten, vorgegebenen Menge an korrespondierenden Antikörpern reagieren. Dabei konkurrieren die markierten und nicht-markierten Antigene um die zahlenmäßig begrenzten "Antikörper-Plätze". Je geringer die Menge an nicht-markierten, zu bestimmenden Antigenen ist, desto mehr mit einem Enzym markierte Antigene werden an die Antikörper gebunden.

Die Antikörper dabei an einen festen Träger gebunden sein. Man entfernt nach der oben beschriebenen Umsetzung die anderen Reaktanten, beispielsweise durch einfaches Spülen. Übrig bleibt der Träger mit den daran fixierten Antikörpern, an welche die Antigene (Bei dem angegebenen Beispiel handelt es sich sowohl um markierte als auch nichtmarkierte Antigene) gebunden bzw. angelagert sind. Die Menge an markierten Antigenen, die an die Antikörper gebunden sind, wird mit Hilfe der Luciferin-Derivate bestimmt. Man kann daraus die Menge an nicht-markierten Antigenen errechnen. In diesem Fall stellt der Ligand somit diejenige Art von biochemisch aktiver Substanz dar, die man zu bestimmen wünscht.

Es ist jedoch auch möglich, das Enzym mit einem Liganden zu konjugieren, der selbst nicht bestimmt werden soll. Dies heißt, daß der markierte Ligand nicht diejenige Art von biochemisch aktiver Substanz darstellt, die man zu bestimmen wünscht. Vielmehr wird der markierte Ligand mit dem entsprechenden, zu bestimmenden biochemischen Gegenstück umgesetzt. Auf diese Weise erhält man mittels des markierten Liganden Aussagen über das genannte Gegenstück.

Die beschriebene Art der Detektion mit Hilfe der Luciferin-Derivate kann jedoch nicht nur bei Enzym-Immunoassays angewendet werden. Vielmehr können die erfindungsgemäßen Verbindungen der allgemeinen Formel (I) sowie Luciferinmethylester und O-Methylluciferin auch als Substrate zur Detektion bei Blot-Verfahren (Western blot) und bei Nukleinsäurehybridisierungen Anwendung finden.

Das erfindungsgemäße Detektionssystem kann allgemein zur Detektion von all solchen Liganden eingesetzt werden, die mit einem entsprechenden Enzym markiert werden können, und die mit einer damit korrespondierenden biochemischen Substanz unter Anlagerung reagieren können. Es ist dabei auch möglich, sich zusätzlicher Zwischenglieder zu bedienen, um die eigentlich zu bestimmende biochemische Substanz an den Liganden binden zu können. So kann man dazu ein entsprechendes Zwischenglied an die biochemische Substanz anlagern, wobei das Zwischenglied wiederum in der Lage ist, sich mit dem Liganden unter Anlagerung zu verbindern.

Die bei den Enzym-Immunoassays zur Anwendung kommenden Enzym-Konjugate können nach bekannten Methoden hergestellt werden, die beispielsweise in folgenden Druckschriften beschrieben sind: J. Carlsson et al, Biochem. J. 173, 723—737 (1978) und H. Tae, Meth. Enzymol. 91, 580—609 (1983).

Als Enzyme setzt man erfindungsgemäß solche ein, die aus einem Luciferin-Derivat, worunter nachstehend auch der Luciferinmethylester sowie O-Methylluciferin verstanden wird, die Verbindung Luciferin freisetzen können.

Als geeignete Enzyme können genannt werden: Amidasen, Aminoacylase I, Esterasen, Carboxypeptidase A und B, Kininase II, Arylsulfatase, alkalische und saure Phosphatase, Lipasen, Acetylesterase, Nukleotidasen, Phospholipase A—D, α- und β-Glucosidasen, α- und β-Amylasen und Nukleasen.

So spaltet beispeilsweise Carboxylesterase (EC 3.1.1.1) die Verbindungen der allgemeinen Formel (I), worin $R^2$ ein Wasserstoffatom und $R^1$ eine Alkenyl- oder Alkoxygruppe bedeuten, in Luciferin und Alkohol. So entsteht beispielsweise aus Luciferinmethylester Luciferin und Methylalkohol.

Carboxypeptidase A (EC 3.4.17.1) spaltet die Luciferin-Derivate der allgemeinen Formel (I), worin $R^2$ ein Wasserstoffatom und $R^1$ eine Aminosäure bedeuten, in Luciferin und die betreffende Aminosäure So

entsteht beispielsweise aus Luciferylphenylalanin, Luciferylglycin oder Luciferylmethionin Luciferin sowie Phenylalanin, Glycin oder Methionin.

Die Carboxypeptidase A ist dabei hinsichtlich der Aminosäure unspezifisch.

Die Carboxypeptidase B (EC 3.4.17.2) spaltet solche Verbindungen der allgemeinen Formel (I), worin $R^2$ ein Wasserstoffatom und $R^1$ Arginin bzw. Lysin bedeuten. Es entsteht dabei Luciferin und Arginin bzw. Lysin.

α und β-Amylasen (EC 3.2.1.1) und EC 3.2.1.2) spalten die Verbindungen der allgemeinen Formel (I), worin $R^1$ eine Hydroxygruppe und $R^2$ ein Disaccharid bedeuten, in das entsprechende Disaccharid und Luciferin.

Kininase II (EC 3.4.15.1) spaltet die Verbindungen der allgemeinen Formel (I), worin $R^2$ ein Wasserstoffatom und $R^1$ ein Dipeptid bedeuten, in das entsprechende Peptid und Luciferin. Steht $R_1$ für andere Oligopeptide, setzt man microbielle Proteinasen (EC 3.4.21.14 bzw. EC 3.4.22.-)ein. Lipasen (EC 3.1.1.-) spalten die Verbindungen der allgemeinen Formel (I), worin $R^1$ eine Hydroxygruppe und $R^2$ einen

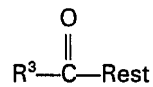

bedeuten, in die ensprechende Säure und Luciferin.

Nukleotidasen (EC 3.1.3.-) sowie Nukleasen spalten die Verbindungen der allgemeinen Formel (I), worin $R^1$ eine Hydroxygruppe und $R^2$ ein Nukleotid bedeuten, in das entsprechende Nukleotid und Luciferin.

Acetylesterase (CE 3.1.1.6) spaltet die Verbindungen der allgemeinen Formel (I), worin $R^1$ ein Hydroxygruppe und $R^2$ einen $CH_3$—CO—Rest bedeuten, in Essigsäure und Luciferin.

Amidasen spalten Luciferinamid in Luciferin und Ammoniak. Aminoacylase spaltet Luciferylaminosäuren in Luciferin und die entsprechende Aminosäure.

Arylsulfatase (EC 3.1.6.1) spaltet die Verbindungen der allgemeinen Formel (I), worin $R^1$ eine Hydroxygruppe bedeutet und $R^2$ eine $HSO_3$-Gruppe darstellt in Luciferin und $H_2SO_4$.

Alkalische und saure Phosphatase (EC 3.1.3.1) spalten die Verbindungen der allgemeinen Formel (I), worin $R^1$ eine Hydroxygruppe und $R^2$ eine $H_2PO_3$-Gruppe bedeutet in Luciferin und $H_3PO_4$.

α/β-Glucosidasen (EC 3.2.1.20 und EC 3.2.1.21) spalten die Verbindungen der allgemeinen Formel (I), worin $R^1$ eine Hydroxygruppe und $R^2$ einen Glucoserest bedeutet, in die entsprechende α/β-Glucose und Luciferin.

Setzt man im erfindungsgemäßen Immunoassay Verbindungen der allgemeinen Formel (I) ein, worin $R^1$ einen anderen Rest als eine Hydroxygruppe und $R^2$ einen anderen Rest als ein Wasserstoffatom bedeuten, dann muß man zwei der oben genannten Enzyme einsetzen, um aus dem Derivat Luciferin freizusetzen.

Mit den oben beschriebenen Enzymen ist es somit möglich aus einem erfindungsgemäßen Luciferin-Derivat einschlielich des O-Methylluciferin sowie des Luciferinmethylesters Luciferin freizusetzen. Die erfindungsgemäß eingesetzten Verbindungen dienen somit als Substrate zur Detektion von Liganden, die mit Enzymen markiert sind. Derartige mit Enzymen markierte Liganden können in den verschiedensten Anwendungsgebieten, beispielsweise bei Immunoassays, eingesetzt werden.

Das freigesetzte Luciferin ist noch in geringster Konzentration mit dem Enzym Luciferase des Leuchtkäfers Photinus pyralis nachweisbar.

Beim Umsatz von Luciferin mit dem Enzym Luciferase findet folgende Reaktion statt:

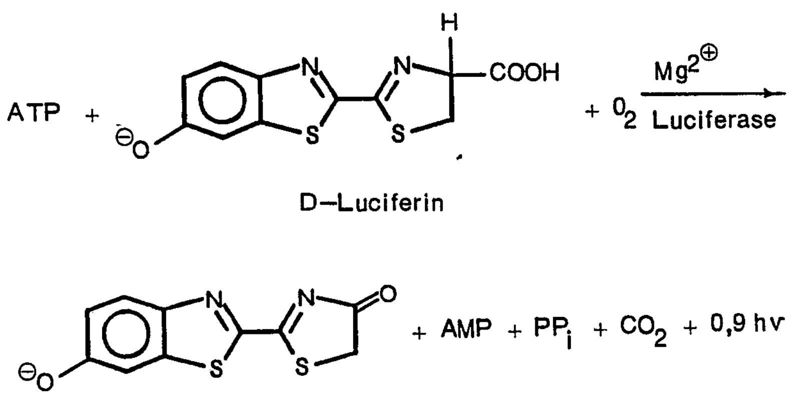


D—Luciferin

Oxyluciferin

Bei dieser Umsetzung wird ATP verbraucht. Photonen mit einer Wellenlänge von 562 nm werden emittiert. Es handelt sich somit um eine Biolumineszenz. Das emittierte Licht bestimmt man luminometrisch.

Die Einzelheiten der oben beschriebenen Umsetzung sind bekannt und unter anderem beschrieben in:

Luminometrie von K. Wulff in Methods of Enzymatic Analysis, Vol. 1 (H. U. Bergmeyer, Herausg.), Sieten 340—368, Verlag Chemie, Weiheim, Bergstraße (1983).

Durch den Umsatz des freigesetzten Luciferins mit der Luciferase wird die Empfindlichkeit der Detektion der markierten Liganden gestiegert. Dies heißt mit anderen Worten, daß erfindungsgemäß ein besseres Detektionssystem zur Verfügung gestellt wird.

Erfindungsgemäß werden somit diejenigen Enzyme, die aus einem Luciferin-Derivat Luciferin freisetzen können, als Marker verwendet. Diese Enzyme können mit den jeweiligen Liganden, wobei es sich um Antikörper, Antigene, Haptene etc. handeln kann, nach bekannten Verfahren konjugiert werden.

Diese Marker-Enzyme können in allen bisher bekannten, beispeilsweise in den eingangs genannten geschilderten, Enzym-Immunoassays eingesetzt werden, seien es nun homogene oder heterogene Enzym-Immunoassays.

Das bei der Umsetzung dieser Enzym-Konjugate mit Luciferin-Derivaten erhaltene Luciferin wird über die Biolumineszenz mit Hilfe der Luciferase bestimmt.

Die erfindungsgemäßen Verbindungen und der erfindungsgemäße Immunoassay können in der Klinischen Chemie zur Bestimmung von Antikörpern und Antigenen (Hormonen (TSH, Tyroxin, $T_3$, $T_4$), Peptiden, Proteinen, etc.), in der Umweltanalytik (immunologische Bestimmung von Giften (Pflanzenschutzmitteln etc.)), Prostaglandinen, Thromboxanen, monoklonaler Antikörper, inder Lebensmittelchemie und bei allen immunologischen Untersuchungsverfahren eingesetzt werden.

Das erfindungsgemäße Detektionssystem kann ferner beim Blot-Verfahren und bei Nukleisäurehybridiserungen eingesetzt werden.

Die erfindungsgemäßen Verbindungen lassen sich wie folgt herstellen.

Die Verbindungen der allgemeinen Formel (I), worin $R^2$ ein Wasserstoffatom bedeutet und $R^1$ eine Alkoxygruppe darstellt, lassen sich herstellen, indem man Luciferin mit einem entsprechenden Alkohol ($R^1$—H) in Anwesenheit von Dicyclohexylcarbodiimid in DMF umsetzt.

Die Verbindungen der allgemeinen Formel (I), worin $R^2$ ein Wasserstoffatom und $R^1$ eine Aminosäure oder ein Oligopeptid bedeuten, kann man herstellen, indem man D-Luciferinhydroxysuccinimidester (vergleiche Beispiel 1) mit der entsprechenden Aminosäure oder dem entsprechenden Oligopeptid umsetzt.

Die Verbindungen der allgemeinen Formel (I), worin $R^1$ eine Hydroxygruppe bedeutet und $R^2$ eine Alkyl- oder Alkenylgruppe, die gegebenenfalls durch einen oder mehrere Phenylrest(e) substituiert ist, kann man folgendermäßen herstellen:

Man setzt Luciferin mit Diazomethan um und erhält so eine Verbindung der Formel (I), worin $R^1$ eine Methoxygruppe bedeutet. Diese Verbindung setzt man mit einem Alkylchlorid (z.B. Butylchlorid) oder Alkenylchlorid zu Verbindungen der allgemeinen Formel (I) um, worin $R^2$ eine $C_1$—$C_{20}$-Alkylgruppe oder eine $C_2$—$C_{20}$-Alkenylgruppe bedeutet. Danach spaltet man die Methylgruppe von $R_1$ enzymatisch mit Carboxylesterase ab und erhält so eine Verbindung der allgemeinen Formel (I), worin $R^1$ eine Hydroxygruppe bedeutet.

Die erfindungsgemäßen Verbindungen der allgemeinen Formel (I), worin $R^2$ eine Arylgruppe bedeutet, erhält man in analoger Weise.

Zur Herstellung der Verbindungen der allgemeinen Formel (I), worin $R^1$ eine Hydroxygruppe und $R^2$ eine Gruppe der allgemeinen Formel (II)

$$R^3-\overset{\overset{\displaystyle O}{\|}}{C}-$$

bedeuten, erhält man ebenfalls in ähnlicher Weise. Jedoch setzt man die entsprechenden Säurechloride ein.

Bei der Synthese der Verbindungen der allgemeinen Formel (I), worin $R^2$ einen Mono- oder Disaccaridrest bedeutet und $R^1$ für eine Hydroxygruppe steht, geht man ebenfalls von Luciferin aus. Nach Schützen der Carboxylgruppe ($R^1$ = $OCH_3$) sestzt man diese geschützte Verbindung mit $^1$Br-Mono- oder Disaccariden um und spaltet anschließend die Methylgruppe des Restes $R^1$ mit Carboxylesterase ab.

Die erfindungsgemäßen Verbindungen, worin $R^1$ eine Hydroxygruppe und $R^2$ einen Nukleotidrest bedeuten, erhält man gemäß folgenden Reaktionsschema:

SCHEMA

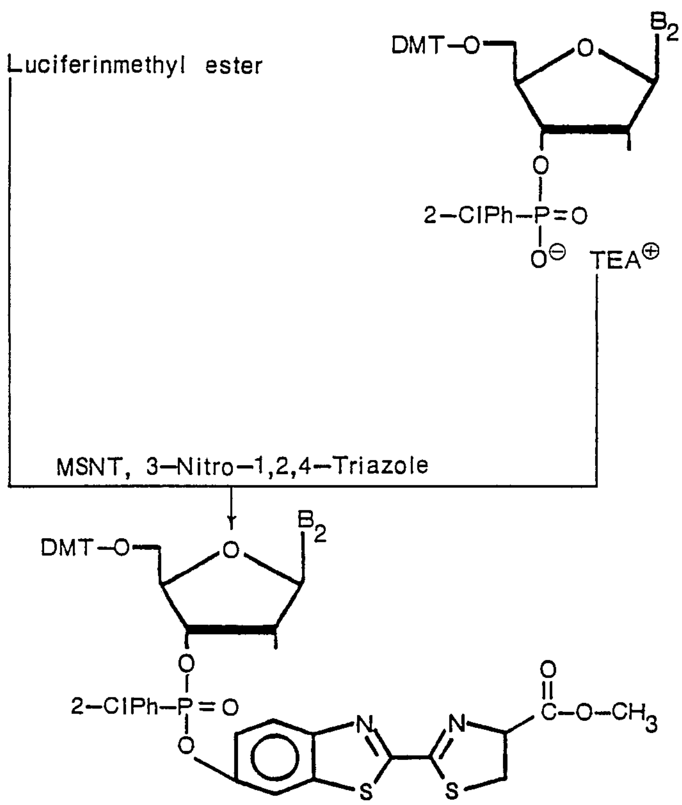


In obigem Schema bedeuten:
$B_2$ = gegebenenfalls geschütze Base;
DMT = 4,4'-Dimethoxytrityl;
TEA = Triethylammonium;
MSNT = 1(Mesitylen-2-sulfonyl)-3-nitro-1,2,4-triazol
2-ClPh = 2-Chlorphenyl

Bei dieser Synthese geht man somit von Luciferinmethylester aus. Diesen setzt man gemäß obigem Schema mit einem geschützen Nukleotid, beispielsweise in DMF, um. Man kann dabei nicht nur Nukleotide mit einer Phosphatgruppe, sondern auch solche mit zwei oder drei Phosphatgruppe einsetzen, d.h. die entsprechenden Di- oder Triphosphate.

Von der erhaltenen Verbindung spaltet man die Schutzgruppen auf an sich bekannte Weise ab und entfernt danach gewünschtenfalls mit Esterase die Methylgruppe, so daß man eine erfindungsgemäße Verbindung mit $R^1$ = OH erhält.

Als geschütztes Nukleotid kann man beispielsweise $N^6$-Benzoyl-5'-O(4,4'-dimethoxytrityl)-2'-deoxyadenosin-3'-(2-chlorphenyl)-phosphat einsetzen. Auch finden solche Verbindungen Anwendung, bei denen die 2'-Deoxyadenosingruppe beispielsweise durch eine 2'-Deoxyguanisin-, 2'-Deoxythymidin-, 2'-Deoxyuridin-, 2'-Deoxycytidin-, 2'-Guanisin-, 2'-Adenosin-, 2'-Cytidin-, 2'-Thymidin- oder 2'-Uridingruppe ersetzt ist.

Nach obigem Schema kann man auch solche erfindungsgemäße Verbindungen erhalten, worin $R^1$ eine $C_{1-20}$-Alkoxy- oder $C_2$—$C_{20}$-Alkenyloxygruppe bedeutet. Man geht dann nicht von dem Methylester, sondern von dem entsprechenden Ester aus.

Unter einer Alkenylgruppe wird im Rahmen der vorliegenden Anmeldung ein aliphatischer Kohlenwasserstoffrest verstanden, der eine oder mehrere Doppelbindungen aufweist, z.B. Vinyl, Allyl, Propenyl, Butenyl. Eine Alkenyloxy-Gruppe ist eine Alkenylgruppe, die über eine Sauerstoffatom gebunden ist.

Zur Herstellung der erfindungsgemäßen Luciferin-Derivate worin $R^1$ einen Aminosäurerest oder ein Oligopeptid bedeutet und $R^2$ ein Nukleotid darstellt, geht man von den Luciferin-Derivaten aus, bei denen

$R^1$ einen Aminosäurerest oder ein Oligopeptid bedeutet. Anschließend schützt man die funktionellen Gruppen des Restes $R^1$ auf an sich bekannte Weise und setzt dann wie im obigen Schema skizziert um.

Zur Herstellung der erfindungsgemäßen Luciferin-Derivate, worin $R^1$ einenAlkoxy-oder Alkenyloxyrest bedeutet und $R^2$ die angegebenen Bedeutungen besitzt, jedoch nicht für einen Nukleotidrest steht, führt man zuerst den Rest $R^1$ und dann den Rest $R^2$ ein. Man verfährt dabei wie eingangs beschrieben. In ähnlicher Weise verfährt man, wenn $R^1$ einen Aminosäurerest oder ein Oligopeptid bedeutet. Jedoch ist es zweckmäßig, die funktionellen Gruppen des Restes $R^1$ vor der Einführung des Restes $R^2$ auf an sich bekannte Weise zu schützen. Diese Schutzgruppen werden nach Einführung des Restes $R^2$ wieder abgespalten.

Die Verbindung, worin $R^1$ eine Aminogruppe und $R^2$ ein Wasserstoffatom bedeuten, stellt man her, indem man den Luciferinhydroxysuccinimidester mit Ammoniak umsetzt.

Die obigen Umsetzungen führt man zweckmäßigerweise in einem inerten organischen Lösungsmittel wie THF, Dioxan, Pyridin oder DMF durch. Die Reinigung kann mittels HPLC erfolgen.

Die Erfindung wird anhand der nachstehenden Biespiele näher erläutert:

In diesen Beispielen werden folgende Puffer eingesetzt:

A: 0,015 mol/l $Na_2CO_3$ pH 9,5

B: 0,01 mol/l $K_2HPO_4$, 0,015 mol/l NaCl, 0,005 % Tween 20 pH 7,4

C: 2,7 mmol/l $KH_2PO_4$, 6,5 mmol/l $Na_2HPO_4$, 0,137 mol/l NaCl, 0,05 % Tween 20, pH 7,4 (vor Gebrauch werden noch 0,1 % Rinderserumalbumin dazugegeben).

## Beispiele für Immunoassays

### Beispiel A

Assay zur Peptidbestimmung, erläutert an Bradykinin

Eine Antibradykinin-Immunoglobulinlösung (30 µg/ml Puffer A) gibt man in die Vertiefungen einer Mikrotiterplatte und inkubiert über Nacht bei 4°C. Dadurch wird erreicht, daß sich das Antibradykinin-Immunoglobulin an die Mikrotiterplatte "absorbiert". Danach wird 5 x mit Puffer B gewaschen.

Anschließend gibt man 0,1 ml eines Bradykinin-Carboxylesterase-Konjugats (50 µ/ml Puffer C) und 0,1 ml einer Bradykinin-Lösung in verschiedenen Konzentrationen (Bradykinin ist ebenfalls in Puffer C gelöst) in die Vertiefungen der Mikrotiterplatte und inkubiert über Nacht bei 4°C. Dabei konkurrieren das Bradykinin-Enzym-(Esterase)-Konjugat und das reine Bradykinin um das Anti-Bradykinin-Immunoglobulin. Je höher die Konzentration an reinem Bradykinin ist, desto weniger Bradykinin-Enzymkonjugat wird an das Anti-Bradykinin-Immunoglobulin gebunden. Es handelt sich hierbei um einen kompetitiven heterogen Enzym-Immunoassay, bei dem der Antikörper an eine feste Phase gebunden ist.

Anschließend wäscht man 5 x mit Puffer B.

In die Vertiefungen der Mikrotiterplatte werden dann 0,2 ml einer Luciferin-methylesterlösung ($2 \times 10^{-6}$ mol/l in 50 mmol/l Tris/HCl; pH 7,5) gegeben und 1 Stunde bei 37°C inkubiert (Detektionssystem I).

Anschließend entnimmt man 0,1 ml aus den jeweiligen Vertiefungen und gibt zu 0,4 ml eines "Biolumineszenz-Cocktails" Dieser "Biolumineszenz-Cocktail" ist wie folgt zusammengesetzt:

30 mmol/l HEPES (N-2-Hydroxyethylpiperazin-N'-2-ethansulfonsäure)
6 mmol/l $MgCl_2$
6 mmol/l ATP
0,5 mmol/l EDTA
80 µmol/l DTT (Dithiothreitol)
1 µg Luciferase (des Leuchtkäfers Photinus pyralis)

Gesamtvolumen: 0,4 ml; pH 7,75

Es werden dann 10 Sek. lang die Lichtimpulse gemessen.

Die erhaltenen Daten korreliert man mit dem in verschiedenen Konzentrationen eingesetzten Bradykinin und erstellt eine Standardkurve, die in Fig. 1 wiedergegeben ist.

Wünscht man nun, eine unbekannte Bradykininkonzentration zu bestimmen, dann führt man den Immunoassay wie oben beschriebenen durch, bestimmt dann 10 Sek. lang die Lichtimpulse und ermittelt aufgrund dieser Daten zusammen mit der Standardkurve die unbekannte Bradykininkonzentration.

Wie man der Fig. 1 entnehmen kann, kann man mit dem erfindungsgemäßen Immunoassay Bradykinin in Konzentrationen bis hinab zu $2 \times 10^{-13}$ [g/Cavität] bestimmen.

Dieser ist wesentlich empfindlicher als der bisher bekannte, zur Bestimmung von Kininen des Harns eingesetzte Radio-Immunoassay. Bei diesem bekannten Radio-Immunoassay wurde Antiserum gegen synthetisches Bradykinin in Kombination mit markierten $tyr^8$-Bradykinin verwendet. Dieser bekannte Assay ist in der Lage, Bradykinin bis lediglich zu einer Konzentration von $10^{-11}$ [g/Cavität] nachzuweisen. Einzelheiten über den bekannten Radio-Immunoassay finden sich in: The Journal of Laboratory and Clinical Medicin, Vol. 91,5 Seiten 721—728, Mai 1978 "A Sensitive Radioimmuno-Assay Method for Urinary Kinins in Man".

Im obigen Beispiel kann man im Konjugat statt der Carboxylesterase auch Carboxypeptidase A (EC

3.4.17.1), Carboxypeptidase B (EC 3.4.17.2), Arylsulfatase (EC 3.1.6.1) oder alkalische Phosphatase (EC 3.1.3.1) einsetzen.

Das beschriebene Detektionssystem I ersetzt man dann durch folgende:

Detektionssystem II (bei Carboxypeptidase A)
0,2 ml D-Luciferyl-(L)-Phenylalaninlösung
(10 µmol/l in 0,05 mol/l Tris/HCl, 3 g/l LiCl pH 7,5)
1 h bei 37°C inkubieren.

Detektionssystem III (bei Carboxypeptidase B)
0,2 ml D-Luciferyl-(L)-$N^{\alpha}$-arginin-Lösung
(10 µmol/l in 0,05 mol/l Tris/HCl, 0,2 mol/l NaCl, pH 7,8)
1 h bei 37°C inkubieren.

Detektionssystem IV (bei Arylsulfatase)
0,2 ml D-Luciferin-O-sulfat-Lösung
(10 µmol/l in 10 mmol/l Natriumacetat, pH 5,0)
1 h bei 37°C inkubieren.

Detektionssystem V (bei alkalischer Phosphatase)
0,2 ml D-Luciferin-O-phosphat-Lösung
(10 µmol/l in 10 mmol/l Diethanolamin, 0,5 mmol/l in $MgCl_2 \cdot 6\ H_2O$, pH 9,8)
1 h bei 37°C inkubieren.

In obigem Beispiel ist die Bestimmung von Bradykinin erläutert. Statt diese Analyten kann man auch andere Analyte bestimmen, beispeilsweise Insulin, Tyroxin $T_3$ und Tyroxin $T_4$.

Beispiel B

Immunoassay zur Bestimmung von Proteinen, beispielsweise Humankallikrein

0,2 ml einer Humanurin-Kallikrein-Lösung (10 µg/ml in Puffer A) gibt man in die Vertiefungen einer Mikrotiterplatte und inkubiert über Nacht bei 4°C. Dabei haftet dieses Antigen aufgrund hydrophober Wechselwirkungen an der Oberfläche der Mikrotiterplatte. Anschließend wäscht man 5 x mit Puffer B (automatisch im Washer).

Parallel zu diesem Ansatz wurden 0,5 ml einer anti-Humankallikrein Immunoglobulin-Lösung (10 ng) mit 0,5 ml einer Humankallikrein-Lösung (in unterschiedlichen Konzentrationen) über Nacht bei 4°C inkubiert.

Anschließend gibt man jeweils 0,2 ml letzteren Inkubationsansatzes in die beschichteten Vertiefungen der Mikrotiterplatten und inkubiert 3 Stunden bei 37°C. Dabei binden sich die noch nicht mit Humankallikrein besetzten Antikörper an das die Mikrotiterplatte gebundene Humankallikrein.

Dann gibt man 0,2 ml einer Anti-rabbit Immunoglobulin-alkalischer Phosphatase-Konjugat-Lösung (1 µl Konjugat/ml Puffer C; stammt von der Ziege) in die Vertiefungen und inkubiert 3 Stunden bei 37°C. Dabei bindet sich das Konjugat an die Antigen-Antikörper-Kombination. Das Konjugat kann sich nur dann anlagern, wenn der oben genannte Antikörper vorhanden ist. Je höher die Menge an Antikörper somit ist, desto höher ist auch die Menge an gebundenen Konjugat. Es handelt sich hier um einen indirekten Enzym-Immunoassay für menschliches Harnkallikrein.

Danach gibt man 0,2 ml einer Luciferin-O-phosphat-Lösung ($1 \times 10^{-5}$ mol/l in 10 mol/l Diethanolamin, 0,5 mmol/l, $MgCl_2 \cdot 6H_2O$, pH 9,8) in die Vertiefungen der Mikrotiterplatte und inkubiert 1 Stunde bei 37°C. Nach der Inkubation entnimmt man jeweils 0,1 ml aus den Vertiefungen und pipettiert zu dem im Beispiel A beschriebenen "Biolumineszenz-Cocktail". Man mißt dann 10 sec. lang die Lichtimpulse. (Detektionssystem V).

Je mehr Konjugat an den fester Träger gebunden worden ist, desto mehr Luciferin wird freigesetzt und desto größer ist die Lichtausbeute.

Aufgrund der erhaltenen Daten kann man eine Standardkurve erstellen, die in Fig. 2 gezeigt ist. Wie man sieht, kann man die Konzentration an Humanurin-Kallikrein in einem Bereich von $10^{-15}$ bis $10^{-12}$ [g/Cavität] bestimmen.

Die mit den bisher bekannten Enzym-Immunoassays erreichte Nachweisgrenze lag bei $5 \times 10^{-9}$ [g/Cavität], man vergleiche J. Clin. Chem. Clin. Biochem. Vol. 18, 1980, Seiten 197—208).

Im obigen Beispiel kann man das eingesetzte Enzym, d.h. die alkalische Phosphatase durch Carboxylesterase, Carboxypeptidase A und B sowie Arylsulfatase ersetzen. In diesem Fall finden dann die Detektionssysteme I, II, III bzw. IV Anwendung.

Gemäß dem in diesem Beispiel beschriebenen Immunoassay kann man nicht nur menschliches Harn-Kallikrein, sondern auch Granulocytenelastase vom Schwein, $\alpha_1$-Proteinase-Inhibitor von Schwein, Plasmakallikrein, Cytochrome, Immunoglobulin G, M, E sowie A, Fibrinogen und Fibrinogen-Spaltprodukte (Fibrinopeptide A, B und C) bestimmen.

Beispiel C

Bestimmung von Antikörpern, die auf Humanurin-Kallikreinspezifisch sind

0,2 ml einer Humanurin-Kallikrein-Lösung (10 µg/ml in Puffer A) gibt man in die Vertiefungen einer Mikrotiterplatte und inkubiert über Nacht bei 4°C. Dabei haftet dieses Antigen aufgrund hydrophober Wechselwirkungen an der Oberfläche der Mikrotiterplatte. Anschließend wäscht man 5 x mit Puffer B (automatisch im Washer).

Das Antikörper-enthaltende Serum (z.B. erhalten von Kaninchen) gibt man anschließend in verschiedenen Konzentrationen in die entsprechenden Vertiefungen der Mikrotiterplatte und inkubiert 3 Stunden bei 37°C (das Antiserum wird mit Puffer C verdünnt). Dabei bindet sich der Antikörper an das Antigen und zwar um so mehr, je höher die Konzentration ist. Anschließend wäscht man wiederum 5 x mit Puffer B.

Dann gibt man 0,2 ml einer Anti-rabbit Immunoglobulin-Esterase-Konjugat-Lösung (50 µl Konjugat/ml Puffer C; stammt von der Ziege) in die Vertiefungen und inkubiert 3 Stunden bei 37°C. Dabei bindet sich das Konjugat an die Antigen-Antikörper-Kombination. Das Konjugat kann sich nur dann anlagern, wenn der oben genannte Antikörper vorhanden ist. Je höher die Menge an Antikörper somit ist, desto höher ist auch die Menge an gebundenem Konjugat.

Nach der Inkubation wäscht man erneut 5 x mit Puffer B.

Danach gibt man 0,2 ml einer Luciferin-methylesterlösung ($2 \times 10^{-6}$ mol/l in 50 mmol/l Tris/HCl; pH 7,5) in die Vertiefungen der Mikrotiterplatte und inkubiert 1 Stunde bei 37°C. Nach der Inkubation entnimmt man jeweils 0,1 ml aus den Vertiefungen und pipettiert zu dem im Beispiel A beschriebenen "Biolumineszenz-Cocktail". Man mißt dann 10 Sek. lang die Lichtimpulse.

Je mehr Konjugat an den festen Träger gebunden worden ist, desto mehr Luciferin wird freigesetzt und desto größer ist die Lichtausbeute.

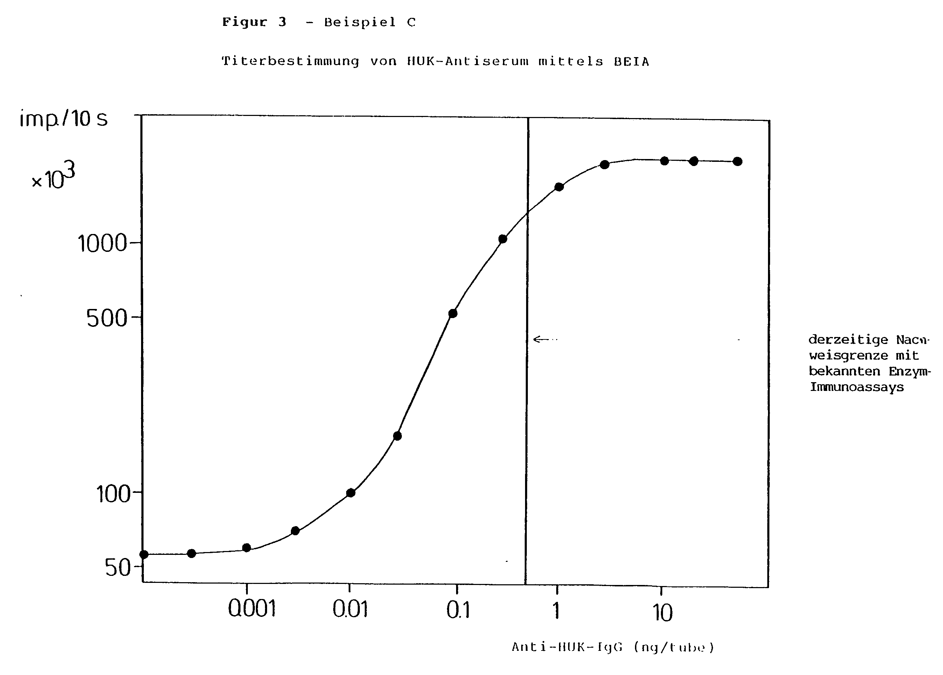

Aufgrund der erhaltenen Daten kann man eine Standardkurve erstellen, die in Fig. 3 gezeigt ist. Wie man sieht, kann man die Konzentration an dem Antikörper für Humanurin-Kallikrein (Anti-HUK-IgG) in einem Bereich von $10^{-12}$ bis $10^{-9}$ [g/Cavität] bestimmen.

Die mit den bisher bekannten Enzym-Immunoassays erreichte Nachweisgrenze lag bei $5 \times 10^{-9}$ [g/Cavität], man vergleiche J. Clin. Chem. Clin. Biochem. Vol. 18, 1980, Seiten 197—208).

Mit diesem Assay können die Antikörper gegen folgende Analyte bestimmt werden: Bradykinin, menschliches Harnkallikrein, Granulocytenelastase von Schwein, $\alpha_1$-Proteinase-Inhibitor vom Schwein, Plasmakallikrein, Cytochrome, Immunoglobulin G, M, E und A, Insulin, Tyroxin $T_3$ und $T_4$, Fibrinogen und Fibrinogen-Spaltprodukte (Fibrinopeptide A, B und C).

Beispiel D

Sandwich-Enzym-Immunoassay für menschliches Harnkallikrein

In die Vertiefungen einer Mikrotiterplatte (Fa. Dynatech) gibt man immunselektiertes Anti-Huk-IgG von der Ziege (10 µg/ml in Puffer A; 0,2 ml/Cavität) und inkubiert über Nacht bei 4°C. Anschließend wäscht man die Platte 5 Mal mit Puffer B und pipettiert 0,2 ml einer Lösung von menschlichem Harnkallikrein (in Puffer C) in die Vertiefungen und inkubiert 24 h bei 4°C. Nach erneutem Waschen mit Puffer B inkubiert man 24 h mit Anti-HUK-IgG vom Kaninchen (10 µg/ml Puffer C). Danach wäscht man 5 x mit Puffer B und inkubiert weitere 24 h mit Anti-Kaninchen-IgG-Enzym-Konjugat (0,2 ml Puffer C), wobei man als Enzym Carboxylesterase, Carboxypeptidase A und B, Arylsulfatase bzw. alkalische Phosphatase einsetzt. Zur Bestimmung des gebundenen Anti-Kaninchen-IgG-Enzym-Konjugats setzt man je nach Enzym das Detektorssystem I bis V ein.

Beispiele für weitere Anwendung des erfindungsgemäßen Detektionssystems

Beispiel E

Durchführung eines Blot-Verfahrens (Western-Blot- konkretes Beispiel: Protein-Inhibitor gegen Schweineleukocytenelastase)

Wie in der Literatur beschrieben (1) wird zuerst eine SDS-Elektrophorese mit dem Hemmstoff durchgeführt. Dann wird das Protein vom Acrylamidgel auf eine Nitrocellulose-Folie übertragen (1). Anschließend erfolgt die Sichtbarmachung des "geblotteten" (transferierten) Inhibitors wie folgt: Die Nitrocellulose-Folie wird 24 h bei Raumtemperatur mit einem Antikörper-alkalische Phosphatase Konjugat in Puffer B inkubiert (der Antikörper ist gegen den Inhibitor gerichtet). Danach wird mit Puffer A gewaschen und die Nitrocellulose-Folie in einen Trog gelegt, der mit dem Biolumineszenz-Cocktail und 10 µmol/l D-Luciferin-O-Phosphat benetzt ist. (Die Viskosität des Biolumineszenz-Cocktails wurde mit einem Gelierungsmittel (z.B. Agar erhöht)). Das Ganze wird unter Lichtausschluß auf einen hochempfindlichen S/W-Film (Kodak TRi-X pan professional film) gelegt und 2 h der Strahlung bei Raumtemperatur ausgesetzt. Dann wird entwickelt. Die Lage des Inhibitors kann durch die Schwarzfärbung bestimmt werden.

Literatur für Blot-Verfahren:

[1]Towbin et al (1979) Proc. Natl. Acad. Sci. USA 76, 4350.

Literatur für Nukleinsäure-Hybridisierung:

Southern E. M. (1980) Meth. Enzymol. 68, 152

Alwine et al (1980) Meth. Enzymol. 68, 220.

Puffer A: 10 mmol/l $KH_2PO_4$, 15 mmol/l NaCl, 0,05 g/l Tween 20 pH 7,4

Puffer B: 1,5 mmol/l $KH_2PO_{43} \cdot H_2O$, 0,14 mol/l NaCl, 0,5 g/l Tween, 20,2 mg/ml Rinderserumalbumin, pH 7,4.

Beispiel F

Durchführung eines Nukleinsäure-Hybridisierungs-Verfahrens (Southern-Blot- konkretes Beispiel: pBR322 Hybridisierung)

pBR322 DNA wird wie in Literatur (2) beschrieben aus E. coli isoliert und im Acrylamidgel elektrophoretisch getrennt. Dann erfolgt eine Transfer der DNA auf Nitrocellulose wie in Literatur (2) und (3) beschrieben. Danach wird die Hybridisierung auf Nitrocellulose wie folgt durchgeführt:

Die Nitrocellulose wird wie in Literatur 2 und 3 beschrieben behandelt. Dann wird nicht wie sonst üblich radioaktiv markierte pBr322 DNA zur Hybridisierung verwendet, sondern eine pBR322 DNA, die über Nick-Translation mit Biotin markiert wurde (Vorschrift: Bethesda Research Laboratories Kit oder Lit. 2; beschreibt die Markierung der DNA mit Biotin). Die Nitrocellulose-Folie wird 2 h bei 37°C mit einem Streptavidin-Phosphatase-Konjugat in Puffer B inkubiert (Streptavidin bindet spezifisch an biotinylierte pBR322 DNA). Danach wird mit Puffer A gewaschen und die Nitrocellulose-Folie in einen Trog gelegt, der mit dem

(2) Maniatis et al. (1982) Molecular Cloning, Cold Spring Harber Laboratory

(3) B. D. Hames and S. J. Higgins (1985), Nucelis Acid Hybridization, A Practical Approach, IRL Press, Oxford Biolumineszenz-Cocktail und 10 µmol/l D-Luciferin-O-Phosphat benetzt ist (Biolumineszenz-Cocktail wurde mit Gelierungsmitteln (z.B. Agar) "viskoser gemacht"). Das Ganze wird unter Lichtausschluß auf einen hochempfindlichen S/W-Film (Kodak TRi-X pan professional film) gelegt und 2 h der Strahlung bei Raumtemperatur ausgesetzt. Dann wird entwickelt und die Lage des Inhibitors kann durch die Schwarzfärbung bestimmt werden.

Puffer A: 10 mmol/l $KH_2PO_4$, 15 mmol/l NaCl, 0,05 g/l Tween 20 pH 7,4

Puffer B: 1,5 mmol/l $KH_2PO_4 \cdot H_2O$, 0,14 mol/l NaCl, 0,5 g/l Tween 20, 2 mg/ml Rinderserumalbumin, pH 7,4.

## Herstellungsbeispiele

### Beispiel 1

Herstellung von D-Luciferin-hydroxysuccinimidester (L-O-Su; Zwischenverbindung)

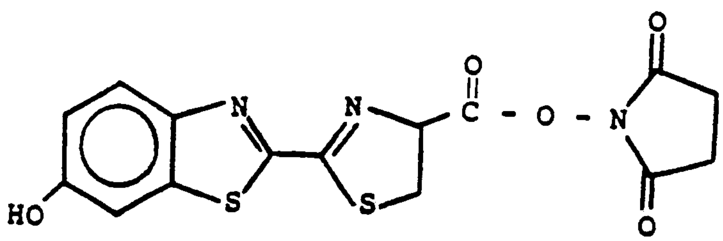

Man löst 30 mg (0,11 mmol) D-Luciferin* und 12,4 mg (0,11 mmol) N-Hydroxysuccinimid bei Raumtemperatur in 1,8 ml Tetrahydrofuran (THF). Zu dieser Lösung gibt man 24,7 mg (0,12 mmol) N,N-Dicyclohexylcarbodiimid (in 0,1 ml THF gelöst) und läßt unter Lichtausschluß 3 Stunden bei Raumtemperatur stehen. Man filtriert den ausfallenden Harnstoff ab, gibt die erhaltene klare Lösung unter Schwenken in 21 ml n-Hexan, saugt den flockigen Niederschlag ab und trocknet ihn im Exsikator. Ausbeute 33 mg (80% d. Th.)

Analyse für $C_{15}H_{11}N_3S_2O_5$ MG: 377,40

| | C | H | N |
|---|---|---|---|
| berechnet: | 49,68 | 3,66 | 10,53 |
| gefunden: | 49,39 | 3,77 | 10,54 |

### Beispiel 2

Reinigung des D-Luciferin-methylesters

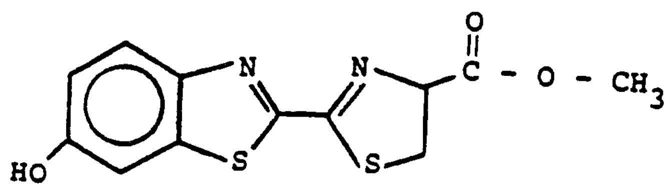

*D-Luciferin: D-(-)-2-(6'-Hydroxy-2'-benzothiazolyl)-$\Delta^2$-thiazolin-4-carbonsäure

Man stellt D-Luciferin-methylester nach E. H. White, J. Amer. Chem. Soc. 102, 3199 (1980) her.

Man reinigt säulenchromatographisch an Kieselgel (Kieselgel 60, Merck 9385), wobei man als Laufmittel Benzol-Ethylacetat (1:1) einsetzt. Ausbeute: 69% d. Th.

Analyse für $C_{12}H_{10}N_2S_2O_3$ MG: 294,35

| | C | H | N |
|---|---|---|---|
| berechnet: | 48,97 | 3,42 | 9,52 |
| gefunden: | 49,59 | 3,77 | 9,41 |

Das Massenspektrum und das UV-Spektrum stehen in Übereinklang mit der Struktur.

## Beispiel 3

Herstellung von D-Luciferyl-L-$N^{\alpha}$-arginin

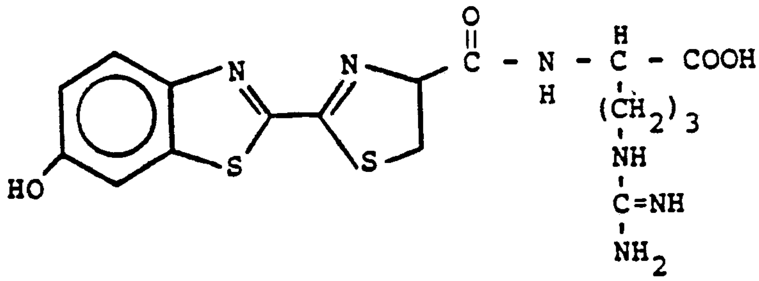

$C_{17}H_{20}N_6S_2O_4$ ; MG 436,52

Man löst 12,25 mg (30 µmol) L-O-Su (hergestellt gemäß Beispiel 1) in 0,3 ml Dioxan und versetzt mit 0,2 ml einer 0,6 mol/l (120 µmol) L-Argininlösung mit pH 7,5 (hergestellt durch Lösen von Arginin in Wasser und Zugabe von 2 N HCl bis pH 7,5). Nach 10 Minuten gibt man 0,015 ml 2 N NaOH zu und stellt den pH der Lösung auf 6 ein. Den Reaktionsansatz läßt man dann 2 Stunden bei Raumtemperatur stehen. Man zieht das Dioxan im Rotationsverdampfer ab, nimmt in Wasser auf, gibt solange 1 mol/l Ammoniak zu, bis alles geöst ist, filtriert und reinigt mittels HPLC (Säule: Hibar EC-RP 8 Merck; Lsg.-A: 0,05 mol/l Ammoniumacetat pH 6,5; Lsg.-B: Methanol für die Chromatographie; A:B = 65:35; Fluß: 1 ml/min.; Druck: ca. 170 bar). Die Ausbeute beträgt 4,81 mg (37% d.Th.).

UV-Maximum: 265,335 mm (Phosphatpuffer pH 7,5).

Fluoreszenzspektrum: Exitation 333 nm; Emission 544 nm (Phosphatpuffer pH 7,8).

Aminosäureanalyse: 0,96 mol Arginin pro mol D-(-)-Luciferin.

## Beispiel 4

Herstellung von D-Luciferyl-L-phenylalanin

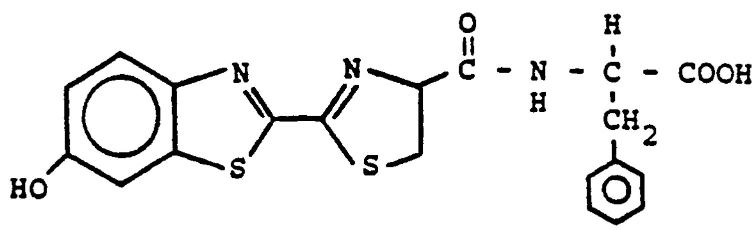

Man löst 4,4 mg (26,5 µmol) L-Phenylalanin in 0,2 ml Dioxan und 0,1 ml Wasser unter Zugabe von 0,06 ml Triethylamin. Diese Lösung gibt man zu 10 mg (26,5 µmol) L-O-Su (hergestellt gemäß Beispiel 1) und läßt 2 Stunden bei Raumtemperatur stehen. Anschließend gibt man 0,01 ml Essigsäure und 1 ml Wasser zu und bewahrt über Nacht bei 4°C auf. Den Niederschlag zentrifugiert man ab, löst ihn in 0,1 mol/l Ammoniak und reinigt mittel HPLC. Die Ausbeute beträgt 6,52 mg (63% d. Th.).

Bruttoformel: $C_{20}H_{17}N_3O_4S_2$; MG: 402,49

UV-Maximum: 270, 335 nm (Phosphatpuffer 7,5)

Fluorenzenzspektren: Exitation 332 nm; Emission 543 nm (Phosphatpuffer 7,5)

Aminosäurenalyse: 1,02 mol Phenylalanin pro mol-D-(-)-Luciferin.

Beispiel 5

Herstellung von D-Luciferyl-L-methionin

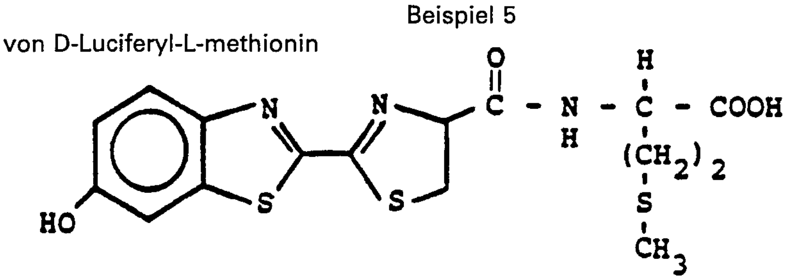

Bruttoformel: $C_{16}H_{17}N_3O_4S_3$; MG: 411,52

Man verfährt wie im Beispiel 4 angegeben, ersetzt jedoch das dort eingesetzte Phenylalanin durch eine äquimolare Menge L-Methionin und erhält so die Titelverbindung.

UV-Maximum: 265, 335 nm (Phosphatpuffer 7,5)

Fluoreszenzspektrum: Exitation 340 nm; Emission 433 nm (Methanol)

Aminosäureanalyse: 0,98 mol Methionin pro mol D-(-)-Luciferin.

Beispiel 6

Herstellung von D-Luciferyl-L-glycin

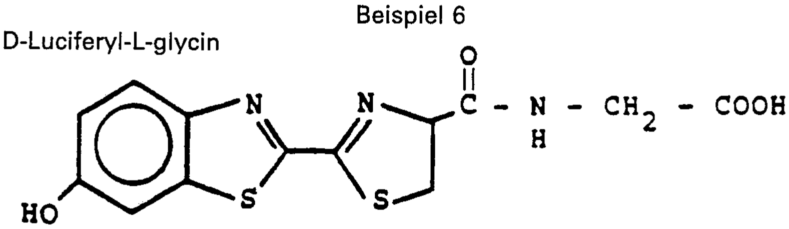

Bruttoformel: $C_{13}H_{11}N_3O_4S_2$; MG 337,37

Man verfährt wie im Beispiel 4, ersetzt jedoch das dort eingesetzte Phenylalanin durch eine äquimolare Menge L-Glycin und erhält so die Titelverbindung.

UV-Maximum: 265, 335 nm (Phosphatpuffer 7,5)

Fluoreszenzspektrum: Exitation 348 nm; Emission 435 nm (Methanol)

Aminosäureanalyse: 0,94 mol Glycin pro mol D-(-)-Luciferin.

Beispiel 7

Herstellung von D-Luciferin-O-sulfat

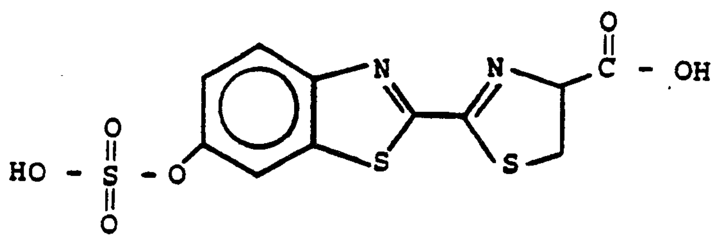

Man löst 20 mg (17 µmol) D-Luciferin in 0,6 ml trockenem Pyridin. Diese Lösung gibt man zu 16 mg (100 µmol) Pyridin-$SO_3$-Komplex. Man läßt 2 Stunden bei Raumtemperatur unter Lichtausschluß stehen und reinigt mittels HPLC wie im Beispiel 3 beschrieben. Die Ausbeute beträgt 37% d.Th.

UV-Maximum: 250, 290 nm (Phosphatpuffer 7,5)

Fluoreszenzspektrum: Exitation 338 nm; Emission 412 nm (Phosphatpuffer pH 5,0)

Bruttoformel: $C_{11}H_8N_2O_6S_3$; MG: 360,39.

Beispiel 8

Herstellung von D-Luciferin-O-phosphat

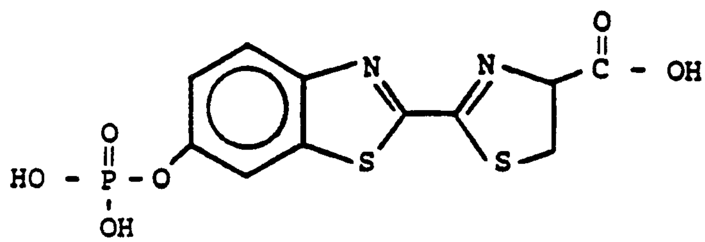

Man löst 50 mg (180 µmol) D-Luciferen in 2 ml Wasser und versetzt mit 350 mg (8,75 mmol) Magnesiumoxid. Zu der gekühlten Suspension tropft man langsam eine Lösung von 92 mg (600 µmol) Phosphoroxychlorid in 1 ml Tetrachlorkohlenstoff. Man läßt anschließend 30 Minuten bei Raumtemperatur stehen, filtriert und neutralisiert mit Essigsäure. Nach Einengen reinigt man mittels HPLC wie im Beispiel 3 beschrieben. Die Ausbeute beträgt 29% d. Th.

UV-Maximum: 260, 315 nm (Phosphatpuffer pH 7,5)

Fluoreszenzspektrum: Exitation 345 nm; Emission 442 nm (Phosphatpuffer pH 7,5)

Bruttoformel: $C_{11}H_9N_2O_6S_2P_1$: MG 359,39

Beispiel 9

Darstellung von D-Luciferin-amid

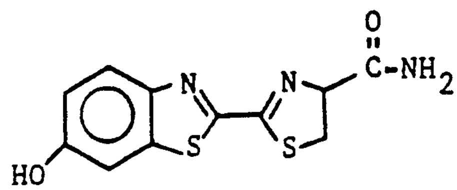

Man löst 12,25 mg (30 µmol) L-O-Su (hergestellt gemäß Beispiel 1) in 0,3 ml Dioxan und versetzt mit 0,2 ml einer 0,6 mol/l [(129 µmol) Ammoniak-Lösung. Den Reaktionsansatz läßt man dann 2 h unter Stickstoff bei Raumtemperatur stehen. Die Lösung engt man im Rotationsverdampfer bis zur Trockene ein. Den Rückstand nimmt man in wenig verdünntem Ammoniak auf, filtriert und reinigt mit HPLC (Säule: Hibar EC-RP 8 Merck; Lsg. A: 0,05 mol/l Ammoniakacetat pH 6,6; Lsg.-B: Methanol für die Chromatographie; A:B = 65:35; Fluß 1 ml/h; Druck: ca. 170 bar). Die Ausbeute beträgt 7,4 mg (82% d. Th.)

Bruttoformel: $C_{12}H_9N_3S_2O_2$ MG: 279,34

Beispiel 10

Darstellung von D-Luciferin-n-hexanester

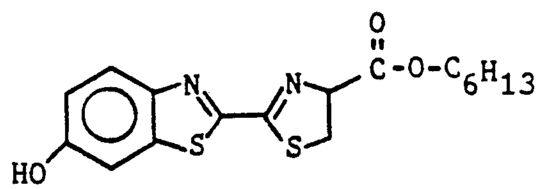

Man löst 40 mg (0,14 mmol) D-Luciferin unter Stickstoff in absolutem Tetrahydrofuran und kühlt auf −15°C. Anschließend versetzt man die Lösung mit 23 µl (0,16 mmol) Triethylamin und 21 µl (0,16 mmol) Chlorameisensäureisobutylester und rührt 30 Min. bei −15°C. Danach tropft man 38 µl (0,03 mmol) n-Hexanol zu, läßt langsam auf Raumtemperatur erwärmen und rührt über Nacht bei dieser Temperatur weiter. Anschließend filtriert man ausgefallenes Triethylamin-hydrochlorid ab, engt das Filtrat ein und nimmt in wenig Methanol auf. Man reinigt mittels HPLC wie im Beispiel 3 beschrieben. Die Ausbeute beträgt 6,6 mg (34% der Th.).

Bruttoformel: $C_{17}H_{20}N_2S_2O_3$ MG: 364,49

Beispiel 11

Darstellung von D-Luciferin-trans-hex-3-en-ester

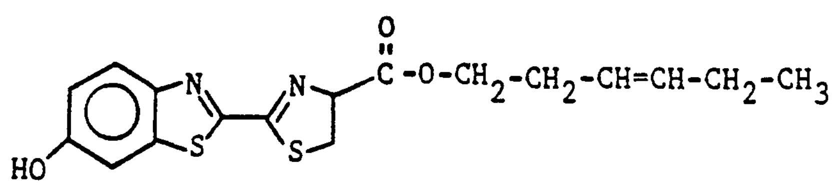

Man löst 40 mg (0,14 mmol) D-Luciferin unter Stickstoff in absolutem Tetrahydrofuran und kühlt auf −15°C. Anschließend versetzt man die Lösung mit 23 µl (0,16 mmol) Triethylamin und 21 µl (0,16 mmol) Chlorameisensäureisobutylester und rührt 30 Min. bei −15°C. Danach tropft man 37 µl (0,3 mmol) trans-3-Hexen-1-ol zu, läßt langsam auf Raumtemperatur erwärmen und rührt über Nacht bei dieser Temperatur weiter. Anschließend filtriert man ausgefallenes Triethylamin-hydrochlorid ab, engt das Filtrat ein und nimmt in wenig Methanol auf. Man reinigt mittels HPLC wie im Beispiel 3 beschrieben. Die Ausbeute beträgt 5,6 mg (29% d. Th.).

Bruttoformel: $C_{17}H_{18}N_2S_2O_3$ MG: 362,47

Beispiel 12

Darstellung von D-Luciferyl-L-Histidyl-L-Leucin

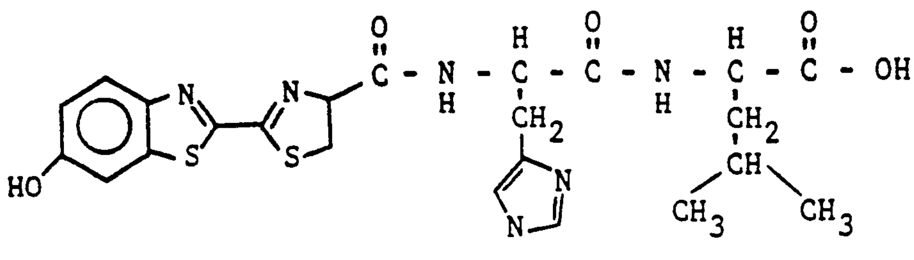

Man löst 7,1 mg (26,5 µmol) L-Histidyl-L-Leucin in 0,2 ml Dioxan und 0,1 ml Wasser unter Zugabe von 0,06 ml Triethylamin. Diese Lösung gibt man zu 10 mg (26,5 µmol) L-O-Su in 0,5 ml Dioxan (hergestellt gemäß Beispiel 1) und läßt 2 h bei Raumtemperatur stehen. Anschließend neutralisiert man mit Essigsäure, gibt 1 ml Wasser zu und bewahrt über Nacht bei 4°C auf. Den Niederschlag zentrifugiert man ab, löst ihn in 0,1 mol/l Ammoniak und reinigt mittels HPLC. Die Ausbeute beträgt 5,3 mg (39% d. Th).

Bruttoformel: $C_{23}H_{27}N_6S_2O_5$ MG: 513,63

Beispiel 13

Darstellung von D-Luciferyl-arginyl-prolyl-prolyl-glycyl-phenylalanyl-serin

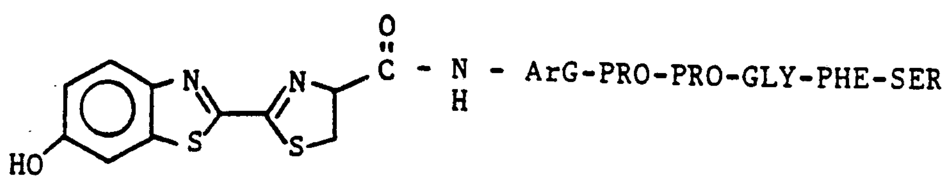

Man löst 17,5 mg (26,5 µmol) L-Arginyl-prolyl-prolyl-glycyl-phenylalanyl-serin in 0,2 ml Dioxan und 0,1 ml Wasser unter Zugabe von 0,06 ml Triethylamin. Diese Lösung gibt man zu 10 mg (26,5 µmol) L-O-Su in 0,5 ml Dioxan (hergestellt gemäß Beispiel 1) und läßt 2 h bei Raumtemperatur stehen. Anschließend neutralisiert man mit Essigsäure, gibt 1 ml Wasser zu und bewahrt über Nacht bei 4°C auf. Den Niederschlag zentrifugiert man ab, löst ihn in 0,1 mol/l Ammoniak und reinigt mittels HPLC. Die Ausbeute beträgt 10,3 mg (42% d. Th.).

Bruttoformel: $C_{41}H_{52}N_{11}S_2O_{10}$ MG 923,08

Beispiel 14

Darstellung von D-Luciferyl-L-ornithin

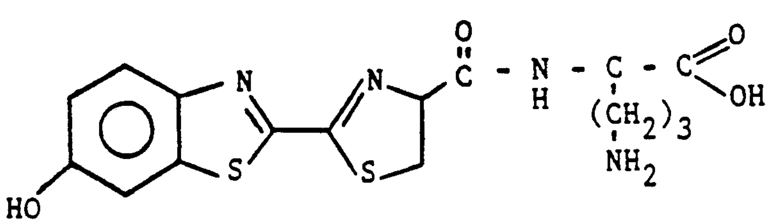

Man löst 13,2 mg (0,053 mmol) N-BOC-L-Ornithin in 0,6 ml Dioxan unter Zugabe von 0,12 ml Triethylamin. Diese Lösung gibt man zu 20 mg (53 mmol) L-O-Su in 0,5 ml Dioxan (hergestellt gemäß Beispiel 1) und läßt 2 h bei Raumtemperatur stehen. Anschließend neutralisiert man mit Essigsäure, git 2 ml Wasser zu und bewahrt über Nacht bei 4°C auf. Den Niederschlag zentrifugiert man ab und trocknet über

Phosphorpentoxid. Anschließend suspendiert man in 1 ml Trifluoressigsäure und rührt unter Wasserausschluß und Stickstoff 1 h bei Raumtemperatur. Danach engt man die Suspension ein, nimmt in wenig verd. Ammoniak auf und reinigt mittels HPLC. Die Ausbeute beträgt 8,8 mg (42% d. Th.).

Bruttoformel: $C_{16}H_{18}N_6S_2O_4$ MG 394,19

Beispiel 15

Darstellung von D-Luciferyl-L-homoarginin

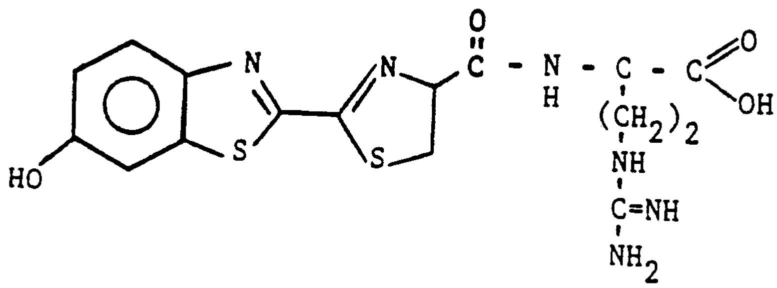

Man löst 12,25 mg (0,030 mmol) L-O-Su (hergestellt gemäß Beispiel 1) in 0,3 ml Dioxan und versetzt mit 0,2 ml einer 0,6 mol/l (120 µmol) L-Homoargininlösung mit pH 7,5 (hergestellt durch Lösen von Homoarginin in Wasser und Zugabe von 2 N HCl bis pH 7,5). Nach 10 Min. stellt man den pH der Lösung auf 6 ein. Den Reaktionsansatz läßt man 2 h bei Raumtemperatur stehen. Danach engt man die Lösung im Rotationsverdampfer ein, nimmt in wenig verd. Ammoniak auf, filtriert und reinigt mittels HPLC. Die Ausbeute beträgt 4,5 mg (33% d. Th.).

Bruttoformel: $C_{16}H_{18}N_6S_2O_4$ MG: 422,35

Beispiel 16

Darstellung von D-Luciferyl-L-citrullin

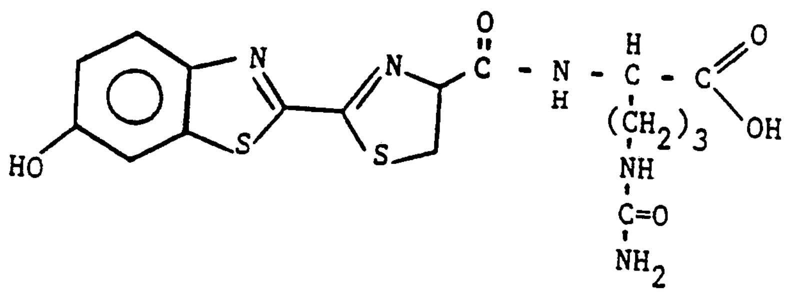

Man löst 12,25 mg (0,030 mmol) L-O-Su (hergestellt gemäß Beispiel 1) in 0,3 ml Dioxan und versetzt mit 21 mg (0,12 mmol) L-Citrullin in 0,2 ml Wasser. Nach 10 Min. stellt man den pH der Lösung auf 6 ein. Den Reaktionsansatz läßt man 2 h bei Raumtemperatur stehen. Danach engt man im Rotationsverdampfer ein, nimmt in wenig verd. Ammoniak auf, filtriert und reinigt mittels HPLC. Die Ausbeute beträgt 6,3 mg (43% d. Th.).

Bruttoformel: $C_{17}H_{20}N_5S_2O_5$ MG: 453,21

Beispiel 17

Darstellung von D-Luciferyl-L-aminobuttersäure

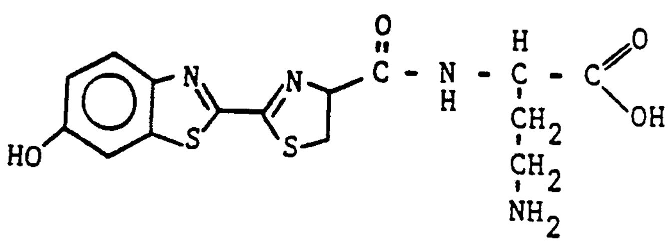

Man löst 5,1 mg (26,5 μmol) Diaminobuttersäure × 2 HCl in 0,2 ml Dioxan und 0,1 ml Wasser unter Zugabe von 0,06 ml Triethylamin. Diese Lösung gibt man zu 10 mg (26,5 μmol) L-O-Su (hergestellt gemäß Beispiel 1) in 0,5 ml Dioxan und läßt 2 h bei Raumtemperatur stehen. Anschließend gibt man 0,01 ml Essigsäure und 1 ml Wasser zu und bewahrt über Nacht bei 4°C auf. Den Niederschlag zentrifugiert man ab, löst ihn in 0,1 mol/l Ammoniak und reinigt mittels HPLC. Die Ausbeute beträgt 5,3 mg (39% d. Th.).

Bruttoformel: $C_{15}H_{16}N_4S_2O_4$ MG: 469,69

Beispiel 18

Darstellung von D-Luciferin-O-hexansäureester

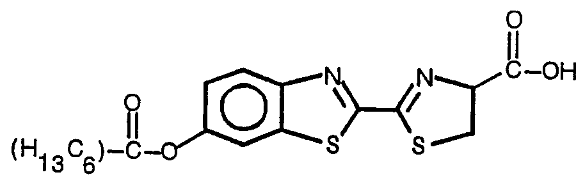

Man löst 30 mg (0,1 mmol) D-Luciferinmethylester in 2 ml trockenem Pyridin. Zu der gekühlten Lösung tropft man langsam eine Lösung von 0,028 ml (0,2 mmol) Hexansäurechlorid in 1 ml trockenem Tetrachlorkohlenstoff. Man läßt anschließend auf Raumtemperatur erwärmen, filtriert und engt im Vakuum ein. Danach wird die Methylgruppe mit Hilfe der Carboxylesterase abgespalten (Rückstand wird in 0,05 mol/l Tris/HCl, pH 8,5 gelöst, vom unlöslichen Material abzentrifugiert, mit 5 U Carboxylesterase versetzt und 2 h unter Stickstoff bei 37°C inkubiert). Anschließend reinigt man mittels HPLC wie im Beispiel 3 beschrieben. Die Ausbeute beträgt 12,3 mg (32% d. Th.).

Bruttoformel: $C_{17}H_{18}N_2S_2O_4$ MG: 378,48

Beispiel 19

Darstellung von D-Luciferen-O-trans-3-hexensäureester

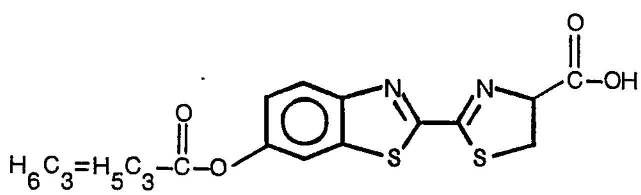

Man löst 30 mg (0,1 mmol) D-Luciferinmethylester in 2 ml trockenem Pyridin. Zu der gekühlten Lösung tropft man langsam eine Lösung von 0,022 ml (0,2 mmol) trans-3-Hexensäurechlorid in 1 ml trockenem Tetrachlorkohlenstoff. Man läßt anschließend auf Raumtemperatur erwärmen, filtriert und engt im Vakuum ein. Danach wird die Methylgruppe mit Hilfe der Carboxylesterase abgespalten (Rückstand wird in 0,05 mol/l Tris/HCl, pH 8,5 gelöst, vom unlöslichen Material abzentrifugiert, mit 5 U Carboxylesterase versetzt und 2 h unter Stickstoff bei 37°C inkubiert). Anschließend reinigt man mittels HPLC wie im Beispiel 3 beschrieben. Die Ausbeute beträgt 7,3 mg (19% d. Th.).

Bruttoformel: $C_{17}H_{16}N_2S_2O_4$ MG: 376,45

Beispiel 20

Darstellung von D-Luciferin-O-palmitylester

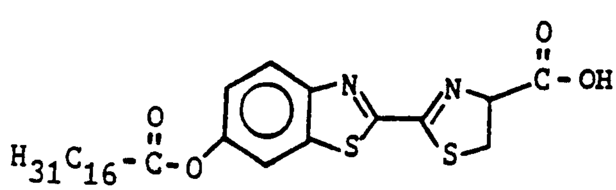

Man löst 30 mg (0,1 mmol) D-Luciferinmethylester in 2 ml trockenem Pyridin. Zu der gekühlten Lösung tropft man langsam eine Lösung von 99 mg (0,2 mmol) Hexadecansäurechlorid in 1 ml trockenem Tetrachlorkohlenstoff. Man läßt anschließend auf Raumtemperatur erwärmen, filtriert und engt im Vakuum

ein. Danach wird die Methylgruppe mit Hilfe der Carboxylesterase abgespalten (Rückstand wird in 0,05 mol/l Tris/HCl, pH 8,5 gelöst, vom unlöslichen Material abzentrifugiert, mit 5 U Carboxylesterase versetzt und 2 h unter Stickstoff bei 37°C inkubiert). Anschließend reinigt man mittels HPLC wie im Beispiel 3 beschrieben. Die Ausbeute beträgt 7,9 mg (15% d. Th.).
Bruttoformel: $C_{27}H_{38}N_2S_2O_4$ MG: 518,75

Beispiel 21

Darstellung von D-Luciferin-O-benzoesäureester

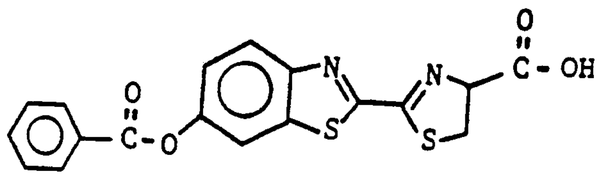

Man löst 30 mg (0,1 mmol) D-Luciferinmethylester in 2 ml trockenem Pyridin. Zu der gekühlten Lösung tropft man langsam eine Lösung von 28 mg (0,2 mmol) Benzoesäurechlorid in 1 ml trockenem Tetrachlorkohlenstoff. Man läßt anschließend auf Raumtemperatur erwärmen, filtriert und engt im Vakuum ein. Danach wird die Methylgruppe mit Hilfe der Carboxylesterase abgespalten (Rückstand wird in 0,05 mol/l Tris/HCl, pH 8,5 gelöst, vom unlöslichen Material abzentrifugiert, mit 5 U Carboxylesterase versetzt und 2 h unter Stickstoff bei 37°C inkubiert). Anschließend reinigt man mittels HPLC wie im Beispiel 3 beschrieben. Die Ausbeute beträgt 9,8 mg (25% d. Th.).
Bruttoformel: $C_{18}H_{12}N_2S_2O_4$ MG: 384,44

Beispiel 22

Darstellung von D-Luciferin-O-naphthansäureester

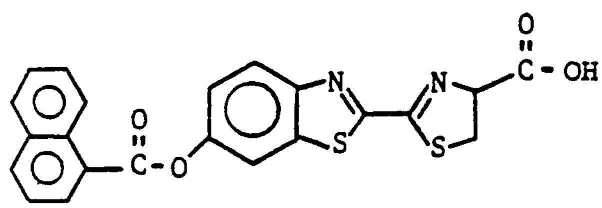

Man löst 30 mg (0,1 mmol) D-Luciferinmethylester in 2 ml trockenem Pyridin. Zu der gekühlten Lösung tropft man langsam eine Lösung von 38 mg (0,2 mmol) 1-Naphthoylchlorid in 1 ml trockenem Tetrachlorkohlenstoff. Man läßt anschließend auf Raumtemperatur erwärmen, filtriert und engt im Vakuum ein. Danach wird die Methylgruppe mit Hilfe der Carboxylesterase abgespalten (Rückstand wird in 0,05 mol/l Tris/HCl, pH 8,5 gelöst, vom unlöslichen Material abzentrifugiert, mit 5 U Carboxylesterase versetzt und 2 h unter Stickstoff bei 37°C inkubiert). Anschließend reinigt man mittels HPLC wie im Beispiel 3 beschrieben. Die Ausbeute beträgt 5,3 mg (12% d. Th.).
Bruttoformel: $C_{22}H_{14}N_2S_2O_4$ MG: 434,50

Beispiel 23

Darstellung von D-Luciferin-o-(C1)glucose

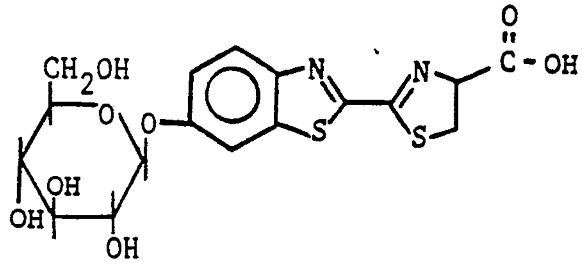

D-Glucose wurde wie in der Literatur (1) beschrieben mit Acetanhydrid zum D-Glucosepyranosepentaacetat acetyliert. Anschließend wird durch Einwirken von HBr das Tetraacetyl-a-D-glucopyranosylbromid dargestellt.

Man löst 60 mg (0,2 mmol) D-Luciferinmethylester in 10 ml Wasser und versetzt mit 280 mg (7 mmol) Magnesiumoxid. Zu der gehkühlten Suspension gibt man langsam 162 mg (0,4 mmol) Tetraacetyl-a-D-glucopyranosylbromid. Man läßt anschließend 48 h bei 40°C stehen, filtriert und neutralisiert mit verdünnten Ammoniak. Nach Einengen wird der Methylrest mit Hilfe der Carboxylesterase abgespalten (Niederschlag wird in 0,05 mol/l Tris/HCl, pH 7,5 gelöst, vom unlöslichen Material abzentrifugiert, mit 5 U Carboxylesterase versetzt und 2 h bei 25°C inkubiert). Die Acetylreste der Tetraacetyl-a-D-glucopyranose werden mittels Ammonolyse abgespalten. Man reinigt mittels HPLC wie im Beispiel 3 beschrieben. Die Ausbeute beträgt 7,2 mg (8% d. Th.).

Bruttoformel: $C_{17}H_{13}N_2S_2O_9$ MG 441,46

(1) Acetylierung von Zuckern, Houben-Weyl-Müller, Methoden der org. Chemie.

### Beispiel 24

Darstellung von D-Luciferin-O-(C1)galactose

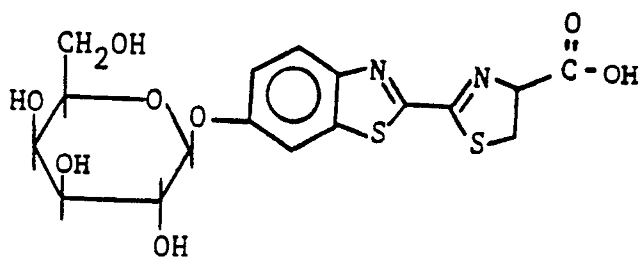

D-Galactose wurde wie in der Literatur (1) beschrieben mit Acetanhydrid zum D-Galactopyranosepentaacetat acetyliert. Anschließend wird durch Einwirken von HBr das Tetraacetyl-a-D-galactopyranosylbromid dargestellt.

Man löst 60 mg (0,2 mmol) D-Luficerinmethylester in 10 ml Wasser und versetzt mit 280 mg (7 mmol) Magnesiumoxid. Zu der gekühlten Suspension gibt man langsam 162 mg (0,4 mmol) Tetraacetyl-a-D-galactopyranosylbromid. Man läßt anschließend 48 h bei 40°C stehen, filtriert und neutralisiert mit verdünntem Ammoniak. Nach Einengen wird der Methylrest mit Hilfe der Carboxylesterase abgespalten (Niederschlag wird in 0,05 mol/l Tris/HCl, pH 7,5 gelöst, vom unlöslichen Material abzentrifugiert, mit 5 U Carboxylesterase versetzt und 2 h bei 25°C inkubiert). Die Acetylreste der Tetraacetyl-a-D-galactopyranose werden mittels Ammonolyse abgespalten. Man reinigt mittels HPLC wie im Beispiel 3 beschrieben. Die Ausbeute beträgt 5,4 mg (6% d. Th.).

Bruttoformel: $C_{17}H_{13}N_2S_2O_9$ MG: 441,46

### Beispiele 25 und 26

Darstellung von D-Luciferin-O-(C1)manose und D-Luciferin-O-(C1)ribose

Obige Verbindungen lassen sich analog zu der Synthese von D-Luciferen-O-($C^1$)glucose (beschrieben im Beispiel 21) hertellen.

### Beispiel 27

Darstellung von D-Luciferin-O-($C^1$)maltose

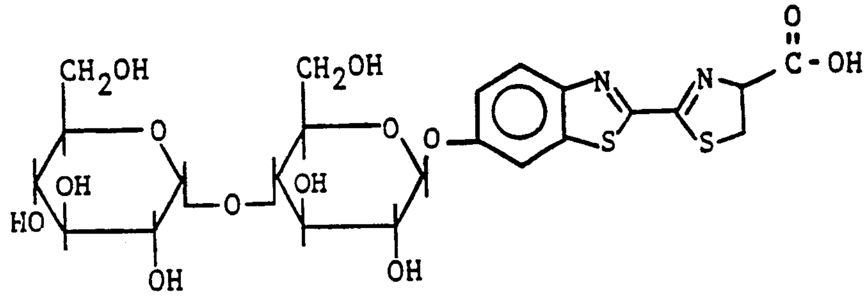

Maltose wurde wie in der Literatur (1) beschrieben mit Acetanhydrid zum Maltose-octaacetat acetyliert. Anschließend wird durch Einwirken von HBr das Heptaacetyl-manosebromid dargestellt.

Man löst 60 mg (0,2 mmol) D-Luciferinmethylester in 10 ml Wasser und versetzt mit 280 mg (7 mmol)

Magnesiumoxid. Zu der gekühlten Suspension gibt man langsam 243 mg (0,4 mmol) Heptaacetyl-manosebromid. Man läßt anschließend 48 h bei 40°C stehen, filtriert und neutralisiert mit verdünnten Ammoniak. Nach Einengen wird der Methylrest mit Hilfe der Carboxylesterase abgespalten (Niederschlag wird in 0,05 mol/l Tris/HCl, pH 7,5 gelöst, vom unlöslichen Material abzentrifugiert, mit 5 U Carboxylesterase versetzt und 2 h bei 25°C inkubiert). Die Acetylreste der Heptaacetyl-manose werden mittels Ammonolyse abgespalten. Man reinigt mittels HPLC wie im Beispiel 3 beschrieben. Die Ausbeute beträgt 6,1 mg (5% d. Th.).

Bruttoformel: $C_{23}H_{27}N_2S_2O_{13}$ MG 600,45

Beispiel 28

Darstellung von D-Luciferin-O-dAMP

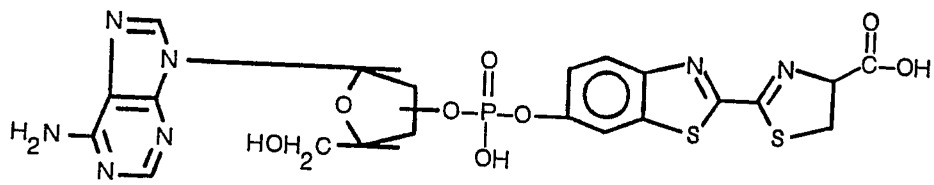

Man löst 60 mg (0,2 mmol) D-Luciferinmethylester in 10 ml wasserfreiem Pyridin und versetzt mit 166 mg (0,2 mmol) $N^6$-Benzoyl-5'-O-(4,4'-dimethoxytrityl)-2'-deoxyadenosin-3'-(2-chlorphenyl)-phosphat. Das Ganze wird in 20 ml trockenem Pyridin wieder aufgenommen, mit 170 mg (0,6 mmol) Mesitylensulfonyltetrazol versetzt und 4 h bei Raumtemperatur inkubiert. Der Reaktionsansatz wird mit 10 ml Wasser anschließend versetzt und zur Trockene einrotiert. Der Rückstand wird in eiskaltem Aceton gelöst, mit 5%-iger $NaHCO_3$-Lösung und Wasser gewaschen. Die organische Phase wird über Natriumsulfat getrocknet und einrotiert. Die Schutzgruppen werden wie in Literatur (2) beschrieben abgespalten und die Substanz wird mittels HPLC gereinigt. Die Ausbeute beträgt 14 mg (12% d. Th.).

Bruttoformel: $C_{20}H_{18}N_7S_2O_8P$ MG: 580,80

Beispiele 29 bis 31

Darstellung von D-Luciferin-O-dGMP, D-Luciferen-O-dCMP und D-Luciferen-O-TMP.

Obige Verbindungen lassen sich analog zum Beispiel 28 herstellen, wobei jedoch $N^6$-Benzoyl-5'-O-(4,4'-Dimethoxytrityl)-2'-deoxyadenosin 3'-(2-Chlorphenyl)-phosphat jeweils durch
$N^2$-Isobutyryl-5'-O-(4,4'-dimethoxytrityl)-2'-deoxyguanosin 3'-(2-Chlorphenyl)-phosphat,
$N^4$-Benzoyl-5'-O-(4,4'-diemethoxytrityl)-2'-deoxycytidin 3'-(2-Chlorphenyl)-phosphat oder
(2) Meth. Enzymol. 65, 610 (1980).
5'-O-(4,4'-Diemethoxytrityl)-2'-thymidin 3'-(2-chlorphenyl)-phosphat eingesetzt wurde.

Aus den in den Beispielen 9 bis 31 beschriebenen Verbindung kann D-Luciferin mit Hilfe der in der nachstehenden Tabelle aufgeführten Enzyme freigesetzt werden.

Tabelle

| Beispiel Nr. | Verwendetes Enzym |
|---|---|
| 9 | Amidase (EC 3.5.1.4 |
| 10 | Carboxylesterase (EC 3.1.1.1) |
| 11 | Carboxylesterase (EC 3.1.1.1) |
| 12 | Angiotensin-convertingenzyme(EC 3.4.15.1) |
| 13 | Gewebskallikrein (EC 3.4.21.35) |
| 14 | Carboxypeptidase A (EC 3.4.17.1) |
| 15 | Cathepsin B (EC 3.4.22.1) |
| 16 | Carboxypeptidase A (EC 3.4.17.1) |
| 17 | Cathepsin B (EC 3.4.22.1) |
| 18 | Carboxylesterase (EC 3.1.1.1) |
| 19 | Carboxylesterase (EC 3.1.1.1) |
| 20 | Phospholipase $A_2$ (EC 3.1.1.4) |
| 21 | Arylesterase (EC 3.1.1.2) |
| 22 | Arylesterase (EC 3.1.1.2) |
| 23 | a/ß-Glucosidase (EC 3.2.1.20/21) |
| 24 | a/ß-Galaktosidase (EC 3.2.1.22/23) |
| 25 | a/ß-Mannosidase (EC 3.2.1.24/25) |
| 26 | 3 -Nucleotidase (EC 3.1.3.6) |
| 27 | a/ß-Amylase (EC 3.2.1.1/2) |
| 28 | 3'-Deoxynucleotidase (EC 3.1.3.34) |
| 29 | 3'-Deoxynucleotidase (EC 3.1.3.34) |
| 30 | 3'-Deoxynucleotidase (EC 3.1.3.34) |
| 31 | 3'-Deoxynucleotidase (EC 3.1.3.34) |

**Patentansprüche für die Vertragstaaten: BE CH DE FR GB IT LI LU NL SE**

1. D-Luciferin-Derivate der allgemeinen Formel (I):

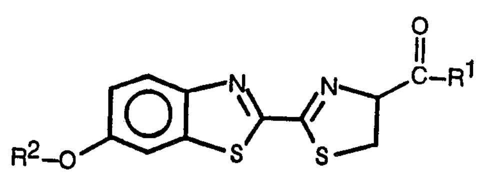

(I)

worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurerest, der über die α-Aminogruppe gebunden ist, oder einen Oligopeptidrest mit bis zu 10 L-aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_{20}$-Alkyl-

oder $C_2$—$C_{20}$-Alkenylgruppe, die gegebenenfalls durch einen oder mehrere Phenylrest(e) substituiert ist, eine Arylgruppe mit 6 bis 18 C-Atomen, eine Gruppe der allgemeinen Formel (II):

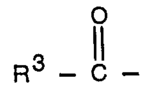
(II)

worin $R^3$ eine gerade oder verzweigte $C_1$—$C_{20}$-Alkyl- oder $C_2$—$C_{20}$-Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist, oder ein glykosidisch angeknüpftes Mono- oder Disaccharid bedeutet;
mit der Ausnahme von D-Luciferen, O-Methylluciferin und D-Luciferin-methylester.

2. Luciferen-Derivate nach Anspruch 1 der allgemeinen Formel (I), worin

$R^1$ eine Hydroxy- oder Aminogruppe, eine $C_{1-6}$-Alkoxy- oder $C_2$—$C_6$-Alkenyloxygruppe, eine natürlich vorkommende Aminosäure oder ein Oligopeptid aus natürlich vorkommenden Aminosäuren mit vorzugsweise bis zu 5 Aminosäureeinheiten bedeutet und

$R^2$ ein Wasserstoffatom, eine $H_2PO_3$- oder $HSO_3$-Gruppe, eine gerade oder verzweigte $C_1$—$C_{20}$-Alkyl- oder $C_2$—$C_{20}$-Alkenylgruppe, die gegebenenfalls durch einen oder mehrere Phenylrest(e) substituiert ist, eine Arylgruppe mit 6 bis 18 C-Atomen, eine Gruppe der allgemeinen Formel (II):

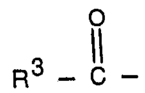
(II)

worin $R^3$ eine gerade oder verzweigte $C_1$—$C_{20}$-Alkyl- oder $C_2$—$C_{20}$-Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist, bedeutet.

3. Luciferin-Derivate nach Anspruch 1 der allgemeinen Formel (I), worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurerest, der über die α-Aminogruppe gebunden ist, oder einen Oligopeptidrest mit bis zu 10 L-aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_6$-Alkyl- oder $C_2$—$C_6$-Alkenylgruppe, die gegebenenfalls durch einen Phenylrest substituiert ist, oder eine Gruppe der allgemeinen Formel (II):

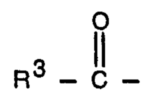
(II)

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

4. Luciferin-Derivate nach Anspruch 1, der allgemeinen Formel (I), worin

$R^1$ eine Hydroxy- oder Aminogruppe, eine $C_1$—$C_6$-Alkoxy- oder $C_2$—$C_6$-Alkenyloxygruppe, ein Aminosäurerest einer natürlich vorkommenden Aminosäure oder ein Oligopeptidrest aus natürlich vorkommenden Aminosäuren mit vorzugsweise bis zu 5 Aminosäureeinheiten bedeutet und

$R^2$ ein Wasserstoffatom, eine $H_2PO_3$- oder $HSO_3$-Gruppe, eine gerade oder verzweigte $C_1$—$C_6$-Alkyl- oder $C_2$—$C_6$-Alkenylgruppe, die gegebenenfalls durch einen Phenylrest substituiert ist oder eine gruppe der allgemeinen Formel (II):

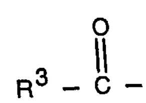
(II)

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

5. Luciferin-Derivate nach Anspruch 1 der allgemeinen Formel (I), worin

$R^1$ eine Hydroxy- oder Aminogruppe, eine $C_{1-6}$-Alkoxygruppe oder eine Gruppe der allgemeinen Formel (III)

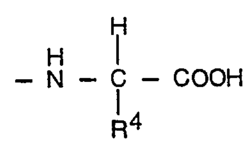
(III)

bedeutet, worin $R^4$ für

$-H$, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2C_6H_5$, 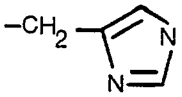, 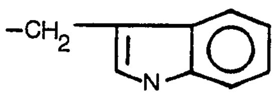, $-CH_2SH$,

$-CH_2CH_2CH_2-NH-C(=NH)-NH_2$ oder $-CH_2CH_2SCH_3$,

steht, oder worin die Gruppe der allgemeinen Formel (III) ein Prolin- oder Hydroxyprolinrest ist, und

$R^2$ ein Wasserstoffatom, eine $C_1$—$C_6$-Alkylgruppe, die gegebenenfalls durch einen Phenylrest substituiert ist, eine Phenyl- oder Naphthylgruppe, eine $H_2PO_3$- oder $HSO_3$-Gruppe oder eine Gruppe der Formel II

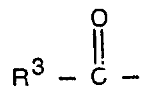 (II)

bedeutet, wobei $R^3$ eine gegebenenfalls durch einen Phenylrest substituierte $C_1$—$C_6$-Alkylgruppe darstellt.

6. Luciferen-Derivate nach Anspruch 1 der allgemeinen Formel (I), worin $R^2$ ein Wasserstoffatom bedeutet, falls $R^1$ einen anderen Rest als eine Hydroxygruppe darstellt, oder worin $R^1$ eine Hydroxygruppe bedeutet, falls $R^2$ einen anderen Rest als ein Wasserstoffatom darstellt.

7. Luciferen-Derivate nach Anspruch 1 der allgemeinen Formel (I), nämlich:
D-Luciferyl-L-$N^{\alpha}$-arginin,
D-Luciferyl-L-phenylalanin,
D-Luciferyl-L-methionin,
D-Luciferyl-L-glycin,
D-Luciferyl-O-sulfat,
D-Luciferyl-O-phosphat.

8. Verwending der Luciferin-Derivate der allgemeinen Formel (I) nach einem der Ansprüche 1 bis 7 und des O-Methyl-luciferins sowie des D-Luciferinmethylesters bei der Bestimmung biochemisch aktiver Substanzen zur Detektion von Liganden, an die unter Bildung eines Ligand-Enzym-Konjugats ein Enzym gekoppelt ist, das in der Lage ist, aus den genannten Luciferin-Derivaten bzw. dem D-Luciferin-methylester sowie dem O-Methylluciferin Luciferin freizusetzen.

9. Verwending nach Anspruch 8 in Immunoassays zur Detektion von Antikörper-, Antigen- oder Hapten-Enzym-Konjugaten, in Blot-Verfahren oder bei Nukleinsäure-Hybridisierungs-Verfahren.

10. Verfahren zur Detektion von Liganden bei der Bestimmung biochemisch aktiver Substanzen, wobei man den Liganden mit einem Enzym unter Bildung eines Ligand-Enzym-Konjugats markiert, dadurch gekennzeichnet, daß man ein Enzym einsetzt, das aus den Luciferin-Derivaten der allgemeinen Formel (I) nach einem der Ansprüche 1 bis 7, aus O-Methylluciferin oder aus D-Luciferinmethylester Luciferin freisetzen kann, mit Hilfe des Enzym-Konjugats aus einem Luciferin-Derivat oder aus D-Luciferinmethylester oder aus O-Methylluciferin Luciferin freisetzt, das freigesetzte Luciferin mit dem Enzym Luciferase des Leuchtkäfers Photinus pyralis umsetzt, die dabei abgestrahlte Lichtmenge quantitativ auswertet, insbesonders luminometrisch, und anhand der erhaltenen Daten die Menge der zu bestimmenden Substanz ermittelt.

11. Verfahren nach Anspruch 10, dadurch gekennzeichnet, daß man als Luciferin freisetzendes Enzym Esterasen, Carboxypeptidase A, Carboxypeptidase B, Arylsulfatase, alkalische und saure Phosphatase, Lipasen, Acetylesterase, Nukleotidasen, Kininase II, α- und β-Glucosidasen, α/β-Amylasen, Amidasen, Aminoacylase I, Phospholipase A—D; Nukleasen und/oder mikrobielle Proteinasen einsetzt.

12. Verfahren nach Anspruch 10 zur Detektion von Antigenen, Haptenen oder Antikörpern, die mit einem Enzym unter Bildung eines Enzym-Konjugats markiert sind, in Immunoassays, dadurch gekennzeichnet, daß man mit Hilfe des Enzym-Konjugats aus einem Luciferin-Derivat bzw. aus dem D-Luciferinmethylester oder dem O-Methylluciferin Luciferin freisetzt, das freigesetzte Luciferin mit dem Enzym Luciferase des Leuchtkäfers Photinus pyralis umsetzt, die dabei abgestrahlte Lichtmenge quantitativ ermittelt, insbesondere luminometrisch, und anhand der erhaltenen Daten die Menge des zu bestimmenden Antigens, Antikörpers bzw. Haptens ermittelt.

**Patentansprüche für den Vertragsstaat: AT**

1. Verfahren zur Herstellung der D-Luciferin-Derivate der folgenden allgemeinen Formel (I):

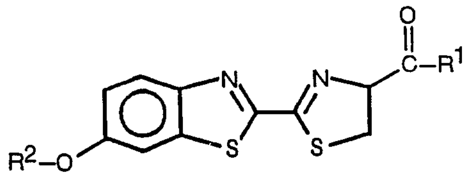

(I)

worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurerest, der über die α-Aminogruppe gebunden ist, oder einen Oligopeptidrest mit bis zu 10 L-aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_{20}$-Alkyl- oder $C_2$—$C_{20}$-Alkenylgruppe, die gegebenenfalls durch einen oder mehrere Phenylrest(e) substituiert ist, eine Arylgruppe mit 6 bis 18 C-Atomen, eine Gruppe der allgemeinen Formel (II):

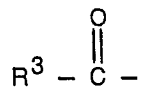

(II)

worin $R^3$ eine gerade oder verzweigte $C_1$—$C_{20}$-Alkyle- oder $C_2$—$C_{20}$-Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist, oder ein glykosidisch angeknüpftes Mono- oder Disaccharid bedeutet;

mit der Ausnahme von D-Luciferin, O-Methylluciferin und D-Luciferin-methylester, dadurch gekennzeichnet, daß man

(a) zur Herstellung der Verbindungen der allgemeinen Formel I, worin $R^2$ ein Wasserstoffatom bedeutet und $R^1$ eine wie oben definierte Alkoxygruppe darstellt, Luciferin mit einem entsprechenden Alkohol ($R^1$—H) in Anwesenheit von Dicyclohexylcarbodiimid in DMF umsetzt,

(b) zur Herstellung der Verbindungen der allgemeinen Formel I, worin $R^2$ ein Wasserstoffatom und $R^1$ einen wie oben definierten Aminosäurerest oder ein Oligopeptid bedeuten, D-Luciferinhydroxysuccinimidester mit der entsprechenden Aminosäure oder dem entsprechenden Oligopeptid umsetzt,

(c) zur Herstellung der Verbindungen der allgemeinen Formel I, worin $R^1$ eine Hydroxygruppe und $R^2$ eine wie oben definierte Alkyl-, Alkenyl- oder Arylgruppe oder eine wie oben definierte Gruppe der allgemeinen Formel II bedeuten, Luciferin mit Diazomethan zu einer Verbindung der allgemeinen Formel I, worin $R^1$ eine Methoxygruppe bedeutet, umsetzt, letztere Verbindung mit einem Alkyl-, Alkenyl- oder Arylchlorid oder einem Säurechlorid von einer Verbindung der oben definierten allgemeinen Formel II umsetzt und die Methylgruppe von $R^1$ enzymatisch mit Carboxylesterase abspaltet,

(d) zur Herstellung der Verbindungen der allgemeinen Formel I, worin $R^1$ eine Hydroxygruppe und $R^2$ einen Mono- oder Disaccharidrest bedeuten, Luciferin nach Schützen der Carboxylgruppe mit einem $^1$Br-Mono- oder Disaccharid umsetzt und anschließend die Methylgruppe des Restes $R^1$ mit Carboxylesterase abspaltet,

(e) zur Herstellung der Verbindungen der allgemeinen Formel I, worin $R^1$ eine Hydroxygruppe oder eine wie oben definierte Alkoxy- oder Alkenyloxygruppe bedeutet und $R^2$ einen wie oben definierten

Nucleotidrest darstellt, einen Luciferinester, wobei es sich um den Luciferinmethylester oder einem dem Rest $R^1$ entsprechenden Ester handelt, mit einem Nucleotid gemäß dem nachstehenden Schema

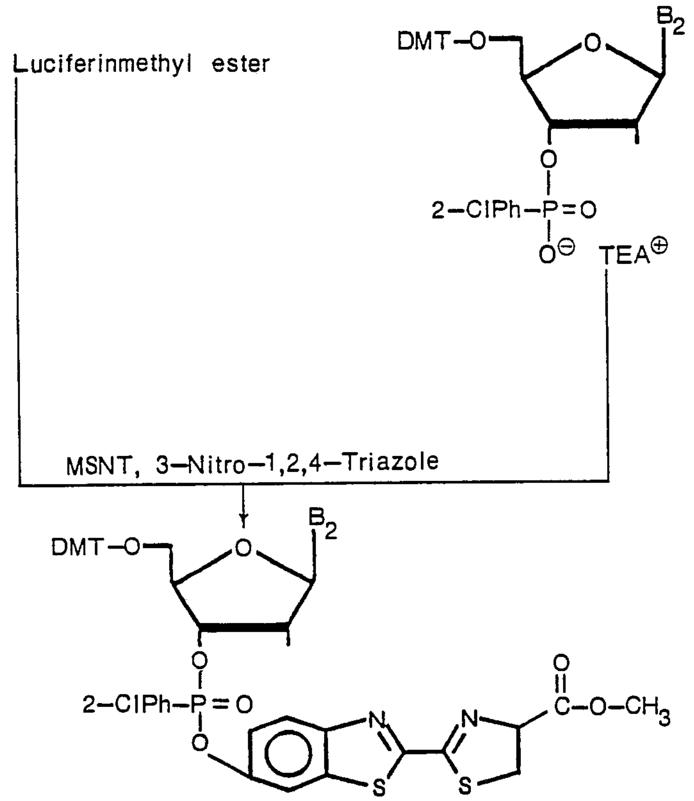


(In obigem Schema bedeuten:
$B_2$ = gegebenenfalls geschützte Base;
DMT = 4,4'-Dimethoxytrityl;
TEA = Triethylammonium;
MSNT = 1(Mesitylen-2-sulfonyl)-3-nitro-1,2,4-triazol
2-ClPh = 2-Chlorphenyl)

umsetzt, von der erhaltenen Verbindung die Schutzgruppen auf an sich bekannte Weise abspaltet und gewünschtenfalls danach im Falle des Methylesters die Methylgruppe mit Esterase abspaltet,

(f) zur Herstellung der Verbindungen der allgemeinen Formel I, worin $R^1$ einen wie oben definierten Aminosäurerest oder ein wie oben definiertes Oligopeptid bedeuten und $R^2$ ein wie oben definiertes Nucleotid darstellt, bei einem Luciferinderivat der allgemeinen Formel I, worin $R^1$ einen Aminosäurerest oder ein Oligopeptid bedeutet, die funktionellen Gruppen dieses Restes $R^1$ auf an sich bekannte Weise schützt und dann gemäß dem in (e) gezeigten Schema umsetzt,

(g) zur Herstellung der Verbindungen der allgemeinen Formel I, worin $R^1$ einen wie oben definierten Alkoxy- oder Alkenyloxyrest bzw. einen Aminosäurerest oder ein Oligopeptid bedeuten und $R^2$ für die oben angegebenen Gruppen oder Reste, jedoch nicht für einen Nucleotidrest, steht, zuerest den Rest $R^1$ wie oben beschrieben einführt und erst dann den Rest $R^2$ einführt,

(h) zur Herstellung der Verbindung der allgemeinen Formel I, worin $R^1$ eine Hydroxygruppe und $R^2$ eine $HSO_3$-Gruppe bedeuten, Luciferen mit einem Pyridin-$SO_3$-Komplex umsetzt,

(i) zur Herstellung der Verbindung der allgemeinen Formel I, worin $R^1$ eine Hydroxygruppe und $R^2$ eine $H_2PO_3$-Gruppe bedeuten, Luciferen mit Phosphoroxychlorid umsetzt, und

(j) zur Herstellung der Verbindung der allgemeinen Formel I, worin $R^1$ eine Aminogruppe und $R^2$ ein Wasserstoffatom bedeuten, Luciferinhydroxysuccinimidester mit Ammoniak umsetzt.

2. Verfahren nach Anspruch 1, dadurch gekennzeichnet, daß man Luciferin-Derivate der allgemeinen Formel I herstellt, worin

$R^1$ eine Hydroxy- oder Aminogruppe, eine $C_1$—$C_6$-Alkoxy- oder $C_2$—$C_6$-Alkenyloxygruppe, eine natürlich vorkommende Aminosäure oder ein Oligopeptid aus natürlich vorkommenden Aminosäuren mit vorzugsweise bis zu 5 Aminosäureeinheiten bedeutet und

$R^2$ ein Wasserstoffatom, eine $H_2PO_3$- oder $HSO_3$-Gruppe, eine gerade oder verzweigte $C_1$—$C_{20}$-Alkyl-

oder $C_2$—$C_{20}$-Alkenylgruppe, die gegebenenfalls durch einen oder mehrere Phenylrest(e) substituiert ist, eine Arylgruppe mit 6 bis 18 C-Atomen, eine Gruppe der allgemeinen Formel (II):

$$R^3-\overset{\overset{\displaystyle O}{\|}}{C}- \qquad (II)$$

worin $R^3$ eine gerade oder verzweigte $C_1$—$C_{20}$-Alkyl- oder $C_2$—$C_{20}$-Alkenylgruppe, die gegebenenfalls durch einen Phenylrest substituiert ist, oder eine $C_6$—$C_{18}$-Arylgruppe darstellt, oder einen natürlich vorkommenden Nucleotidrest mit 1 bis 3 Phosphatgruppen, der über die Phosphatgruppe(n) angeknüpft ist, bedeutet.

3. Verwending nach Anspruch 1, dadurch gekennzeichnet, daß man Luciferin-Derivate der allgemeinen Formel I herstellt, worin

$R^1$ eine Hydroxy- oder Aminogruppe, eine gerade oder verzweigte $C_1$—$C_{20}$-Alkoxy- oder $C_2$—$C_{20}$-Alkenyloxygruppe, einen L-Aminosäurerest, der über die α-Aminogruppe gebunden ist, oder einen Oligopeptidrest mit bis zu 10 L-aminosäureeinheiten, der über die α-Aminogruppe der endständigen Aminosäureeinheit gebunden ist, bedeutet und

$R^2$ ein Wasserstoffatom, eine $H_2PO_3$- oder $HSO_3$-Gruppe, eine gerade oder verzweigte $C_1$—$C_6$-Alkyl- oder $C_2$—$C_6$-Alkenylgruppe, die gegebenenfalls durch einen Phenylrest substituiert ist, oder eine Gruppe der allgemeinen Formel (II):

$$R^3-\overset{\overset{\displaystyle O}{\|}}{C}- \qquad (II)$$

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

4. Verfahren nach Anspruch 1, dadurch gekennzeichnet, daß man Luciferin-Derivate der allgemeinen Formel I herstellt, worin

$R^1$ eine Hydroxy- oder Aminogruppe, eine $C_1$—$C_6$-Alkoxy- oder $C_2$—$C_6$-Alkenyloxygruppe, ein Aminosäurerest einer natürlich vorkommenden Aminosäure oder ein Oligopeptidrest aus natürlich vorkommenden Aminosäuren mit vorzugsweise bis zu 5 Aminosäureeinheiten bedeutet und

$R^2$ ein Wasserstoffatom, eine $H_2PO_3$- oder $HSO_3$-Gruppe, eine gerade oder verzweigte $C_1$—$C_6$-Alkyl- oder $C_2$—$C_6$-Alkenylgruppe, die gegebenenfalls durch einen Phenylrest substituiert ist oder eine gruppe der allgemeinen Formel (II):

$$R^3-\overset{\overset{\displaystyle O}{\|}}{C}- \qquad (II)$$

bedeutet, worin $R^3$ eine $C_1$—$C_6$-Alkyl- oder $C_2$—$C_6$-Alkenylgruppe bedeutet, die gegebenenfalls durch einen Phenylrest substituiert ist.

5. Verfahren nach Anspruch 1, dadurch gekennzeichnet, daß man Luciferen-Derivate der allgemeinen Formel I herstellt, worin

$R^1$ eine Hydroxy- oder Aminogruppe, eine $C_1$—$C_6$-Alkoxygruppe oder eine Gruppe der allgemeinen Formel (III)

$$-\overset{H}{N}-\underset{R^4}{\overset{H}{C}}-COOH \qquad (III)$$

bedeutet, worin $R^4$ für

$-H$, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2\ C_6H_5$, $-CH_2$ 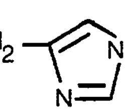 , $-CH_2$ 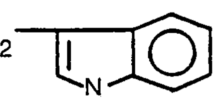 , $-CH_2SH$,

$-CH_2CH_2CH_2$-NH-C(=NH)-$NH_2$ oder $-CH_2CH_2SCH_3$,

steht, oder worin die Gruppe der allgemeinen Formel (III) ein Prolin- oder Hydroxyprolinrest ist, und

$R^2$ ein Wasserstoffatom, eine $C_1$—$C_6$-Alkylgruppe, die gegebenenfalls durch einen Phenylrest substituiert ist, eine Phenyl- oder Naphthylgruppe, eine $H_2PO_3$- oder $HSO_3$-Gruppe oder eine Gruppe der Formel II

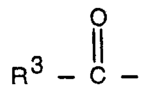

(II)

bedeutet, wobei $R^3$ eine gegebenenfalls durch einen Phenylrest substituierte $C_1$—$C_6$-Alkylgruppe darstellt.

6. Verfahren nach Anspruch 1, dadurch gekennzeichnet, daß man Luciferin-Derivate der allgemeinen Formel I herstellt, worin $R^2$ ein Wasserstoffatom bedeutet, falls $R^1$ einen anderen Rest als eine Hydroxygruppe darstellt, oder worin $R^1$ eine Hydroxygruppe bedeutet, falls $R^2$ einen anderen Rest als ein Wasserstoffatom darstellt.

7. Verwending nach Anspruch 1, dadurch gekennzeichnet, daß man folgende Luciferin-Derivate der allgemeinen Formel I herstellt:

D-Luciferyl-L-$N^{\alpha}$-arginin,
D-Luciferyl-L-phenylalanin,
D-Luciferyl-L-methionin,
D-Luciferyl-L-glycin,
D-Luciferyl-O-sulfat,
D-Luciferyl-O-phosphat.

8. Verwending der Luciferen-Derivate der allgemeinen Formel (I) nach einem der Ansprüche 1 bis 7 und des O-Methyl-luciferins sowie des D-Luciferinmethylesters bei der Bestimmung biochemisch aktiver Substanzen zur Detektion von Liganden, an die unter Bildung eines Ligand-Enzym-Konjugats ein Enzym gekoppelt ist, das in der Lage ist, aus den genannten Luciferin-Derivaten bzw. dem D-Luciferin-methylester sowie dem O-Methylluciferin Luciferin freizusetzen.

9. Verwending nach Anspruch 8 in Immunoassays zur Detektion von Antikörper-, Antigen- oder Hapten-Enzym-Konjugaten, in Blot-Verfahren oder bei Nukleinsäure-Hybridisierungs-Verfahren.

10. Verfahren zur Detektion von Liganden bei der Bestimmung biochemisch aktiver Substanzen, wobei man den Liganden mit einem Enzym unter Bildung eines Ligand-Enzym-Konjugats markiert, dadurch gekennzeichnet, daß man ein Enzym einsetzt, das aus den Luciferin-Derivaten der allgemeinen Formel (I) nach einem der Ansprüche 1 bis 7, aus O-Methylluciferin oder aus D-Luciferinmethylester Luciferin freisetzen kann, mit Hilfe des Enzym-Konjugats aus einem Luciferin-Derivat oder aus D-Luciferinmethylester oder aus O-Methylluciferin Luciferin freisetzt, das freigesetzte Luciferin mit dem Enzym Luciferase des Leuchtkäfers Photinus pyralis umsetzt, die dabei abgestrahlte Lichtmenge quantitativ auswertet, insbesondere luminometrisch, und anhand der erhaltenen Daten die Menge der zu bestimmenden Substanz ermittelt.

11. Verfahren nach Anspruch 10, dadurch gekennzeichnet, daß man als Luciferin freisetzendes Enzym Esterasen, Carboxypeptidase A, Carboxypeptidase B, Arylsulfatase, alkalische und saure Phosphatase, Lipasen, Acetylesterase, Nukleotidasen, Kininase II, α- und β-Glucosidasen, α/β-Amylasen, Amidasen, Aminoacylase I, Phospholipase A—D; Nukleasen und/oder mikrobielle Proteinasen einsetzt.

12. Verfahren nach Anspruch 10 zur Detektion von Antigenen, Haptenen oder Antikörpern, die mit einem Enzym unter Bildung eines Enzym-Konjugats markiert sind, in Immunoassays, dadurch gekennzeichnet, daß man mit Hilfe des Enzym-Konjugats aus einem Luciferin-Derivat bzw. aus dem D-Luciferinmethylester oder dem O-Methylluciferin Luciferin freisetzt, das freigesetzte Luciferin mit dem Enzym Luciferase des Leuchtkäfers Photinus pyralis umsetzt, die dabei abgestrahlte Lichtmenge quantitativ ermittelt, insbesondere luminometrisch, und anhand der erhaltenen Daten die Menge des zu bestimmenden Antigens, Antikörpers bzw. Haptens ermittelt.

**Revendications pour les Etats contractants: BE CH DE FR GB IT LI LU NL SE**

1. Dérivés de D-lucif́erine de formule générale (I):

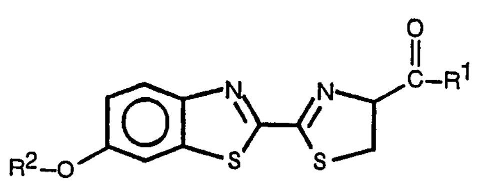

(I)

dans laquelle

$R^1$ représente un groupe hydroxy ou amino, un groupe alcoxy en $C_1$—$C_{20}$ ou alcényloxy en $C_2$—$C_{20}$, linéaire ou ramifié, un reste L-aminoacide, qui est lié par l'intermédiaire du groupe α-amino, ou un reste

oligopeptide, avec jusqu'à 10 unités L-aminoacide, qui est relié par l'intermédiaire du groupe α-amino de l'unité aminoacide terminale, et

$R^2$ représente un atome d'hydrogène, un groupe $H_2PO_3$ ou $HSO_3$, un groupe alkyle en $C_1$—$C_{20}$ ou alcényle en $C_2$—$C_{20}$, linéaire ou ramifié, qui est éventuellement substitué par un ou plusieurs restes phényle, un groupe aryle comportant 6 à 18 atomes de C, un groupe de formule générale (II):

$$R^3-\overset{\overset{\large O}{\|}}{C}- \qquad (II)$$

dans laquelle $R^3$ représente un groupe alkyle en $C_1$—$C_{20}$ ou alcényle en $C_2$—$C_{20}$ linéaire ou ramifié, qui est éventuellement substitué par un reste phényle, ou un groupe aryle en $C_6$—$C_{18}$, un reste nucléotide naturel comportant 1 à 3 groupes phosphate, qui est lié par l'intermédiaire du ou des groupes phosphate, ou un mono ou disaccharide à liaison glycosidique;

à l'exception de la D-luciférine, de l'O-methylluciférine et de l'ester méthylique de la D-luciférine.

2. Dérivés de luciférine selon la revendication 1, de formule générale (I), dans laquelle

$R^1$ représente un groupe hydroxy ou amino, un groupe alcoxy en $C_1$—$C_6$ ou alcényloxy en $C_2$—$C_6$, un aminoacide naturel ou un oligopeptide d'aminoacide naturel, avec de préférence jusqu'à 5 unités aminoacide, et

$R^2$ représente un atome d'hydrogène, un groupe $H_2PO_3$ ou $HSO_3$, un groupe alkyle en $C_1$—$C_{20}$ ou alcényle en $C_2$—$C_{20}$, linéaire ou ramifié, qui est éventuellement substitué par un ou plusieurs restes phényle, un groupe aryle avec 6 à 18 atomes de C, un groupe de formule générale (II):

$$R^3-\overset{\overset{\large O}{\|}}{C}- \qquad (II)$$

dans laquelle $R^3$ représente un groupe alkyle en $C_1$—$C_{20}$ ou alcényle en $C_2$—$C_{20}$ linéaire ou ramifié, qui est éventuellement substitué par un groupe phényle, ou un groupe aryle en $C_6$—$C_{18}$, ou un reste nucléotide naturel avec 1 à 3 groupes phosphate, qui est relié par l'intermédiaire du ou des groupes phosphate.

3. Dérivés de luciférine selon la revendication 1, de formule générale (I), dans laquelle

$R^1$ représente un groupe hydroxy ou amino, un groupe alcoxy en $C_1$—$C_{20}$ ou alcényloxy en $C_2$—$C_{20}$, linéaire ou ramifié, un reste L-aminoacide qui est relié par l'intermédiaire du groupe α-amino, ou un reste oligopeptide avec jusqu'à 10 unités L'aminoacide, qui est relié par l'intermédiaire du groupe α-amino de l'unité aminoacide terminale, et

$R^2$ représente un atome d'hydrogène, un groupe $H_2PO_3$ ou $HSO_3$, un groupe alkyle en $C_1$—$C_6$ ou alcényle en $C_2$—$C_6$ linéaire ou ramifié, qui est éventuellement substitué par un reste phényle ou un groupe de formule générale (II):

$$R^3-\overset{\overset{\large O}{\|}}{C}- \qquad (II)$$

dans laquelle $R^3$ représente un groupe alkyle en $C_1$—$C_6$ ou alcényle en $C_2$—$C_6$, qui est éventuellement substitué par un reste phényle.

4. Dérivés de luciférine selon la revendication 1, de formule générale (I), dans laquelle

$R^1$ représente un groupe hydroxy ou amino, un groupe alcoxy en $C_1$—$C_6$ ou alcényloxy en $C_2$—$C_6$, un reste aminoacide d'un aminoacide naturel ou un reste oligopeptide d'aminoacides naturels, avec de préférence jusqu'à 5 unités aminoacide, et

$R^2$ représente un atome d'hydrogène, un groupe $H_2PO_3$ ou $HSO_3$, un groupe alkyle en $C_1$—$C_6$ ou alcényle en $C_2$—$C_6$, linéaire ou ramifié, qui est éventuellement substitué par un reste phényle, ou un groupe de formule générale (II):

$$R^3-\overset{\overset{\large O}{\|}}{C}- \qquad (II)$$

dans laquelle $R^3$ représente un groupe alkyle en $C_1$—$C_6$ ou alcényle en $C_2$—$C_6$, qui est éventuellement substitué par un reste phényle.

5. Dérivés de luciférine selon la revendication 1, de formule générale (I), dans laquelle

$R^1$ représente un groupe hydroxy ou amino, un groupe alcoxy en $C_1$—$C_6$ ou un groupe de formule générale (III)

$$-\overset{\large H}{N}-\underset{\underset{\large R^4}{|}}{\overset{\overset{\large H}{|}}{C}}-COOH \qquad (III)$$

dans laquelle

$R^4$ représente

$-H$, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2\ C_6H_5$, 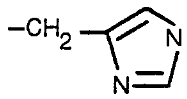 , 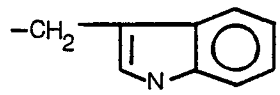 , $-CH_2SH$,

$-CH_2CH_2CH_2-NH-C(=NH)-NH_2$ ou $-CH_2CH_2SCH_3$,

ou dans laquelle le groupe de formule générale (III) est un reste proline ou hydroxyproline, et

$R^2$ représente un atome d'hydrogène, un groupe alkyle en $C_1$—$C_6$ qui est éventuellement substitué par un reste phényle, un groupe phényle ou naphtyle, un groupe $H_2PO_3$ ou $HSO_3$ ou un groupe de formule générale (II):

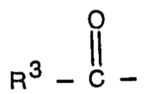 (II)

dans laquelle $R^3$ représente un groupe alkyle en $C_1$—$C_6$ éventuellement substitué par un reste phényle.

6. Dérivés de luciférine selon la revendication 1, de formule générale (I), dans laquelle $R^2$ représente un atome d'hydrogène, dans le cas où $R^1$ représente un autre reste que le groupe hydroxy, ou dans laquelle $R^1$ représente un groupe hydroxy, dans le cas où $R^2$ représente un autre reste qu'un atome d'hydrogène.

7. Dérivés de luciférine selon la revendication 1, de formule générale (I), notamment:

la D-luciféryl-L-N-$^{\alpha}$-arginine,

la D-luciféryl-L-phénylalanine,

la D-luciféryl-L-méthionine,

la D-luciféryl-L-glycine,

le O-sulfate de D-luciférine,

le O-phosphate de D-luciférine.

8. Utilisation des dérivés de luciférine de formule générale (I) selon l'une quelconque des revendications 1 à 7, et de la O-méthylluciférine ainsi que de l'éther méthylique de D-luciférine, pour la détermination de substances ayant une activité biochimique, pour la détection de ligands, avec lesquels est associée une enzyme, avec formation d'un composé conjugué ligand-enzyme, qui est capable de libérer la luciférine à partir des dérivés de luciférine ou de l'ester méthylique de la D-luciférine ainsi que de la O-méthylluciférine mentionnée.

9. Utilisation selon la revendication 8, dans des dosages immunologiques pour la détection de composés conjugués anticorps-, antigène- ou haptène-enzyme, dans des procédés d'absorption ou dans des procédés d'hybridation d'acides nucléiques.

10. Procédé de détection de ligands pour la détermination de substances ayant une activité biochimique, dans lequel on marque le ligand au moyen d'une enzyme, avec formation d'un composé conjugué ligand-enzyme, caractérisé en ce que l'on met en oeuvre une enzyme qui peut libérer la luciférine à partir des dérivés de luciférine de formule générale (I), selon l'une quelconque des revendications 1 à 7, à partir de la O-méthylluciférine ou de l'ester méthylique de la D-luciférine,

à l'aide d'un composé conjugué d'enzyme, on libère la luciférine à partir d'un dérivé de luciférine ou à partir de l'ester méthylique de D-luciférine ou à partir de la O-méthylluciférine,

on fait réagir la luciférine libérée avec l'enzyme luciférase du ver luisant photinus pyralis,

on évalue quantitativement la quantité de lumière émise ici par rayonnement, en particulier par un moyen luminométrique, et

à l'aide des données obtenues, on détermine la substance à déterminer.

11. Procédé selon la revendication 10, caractérisé en ce que comme enzyme libérant la luciférine, on utilise l'estérase, la carboxypeptidase A, la carboxypeptidase B, l'arylsulfatase, les phosphatases alcalines et acides, les lipases, l'acétylestérase, les nucléotidases, la kininase II, les α- et β-glucosidases, les α/β-amylases, les amidases, l'aminoacylase I, la phospholipase A—D; les nucléases et/ou les protéinases microbiennes.

12. Procédé selon la revendication 10, pour la détection d'antigènes, d'haptènes ou d'anticorps, qui sont marqués par une enzyme avec formation d'un produit conjugué d'enzyme, dans des dosages immunologiques, caractérisé en ce que, à l'aide du produit conjugué d'enzyme, on libère la luciférine à partir d'un dérivé de luciférine ou à partir de l'ester méthylique de la D-luciférine ou à partir de la O-méthylluciférine,

on fait réagir la luciférine libérée avec l'enzyme luciférase du ver luisant photinus pyralis,

on évalue quantitativement la quantité de lumière émise ici par rayonnement, en particulier par un moyen luminométrique, et

à l'aide des données obtenues, on évalue la quantité de l'antigène, de l'anticorps ou de l'haptène à déterminer.

**Revendications pour l'Etat contractant: AT**

1. Procédé de préparation de dérivés de D-luciférine de formule générale (I):

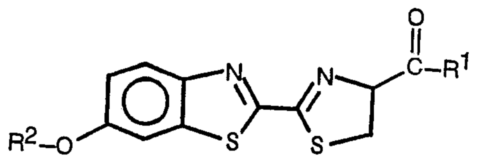

(I)

dans laquelle

$R^1$ représente un groupe hydroxy ou amino, un groupe alcoxy en $C_1$—$C_{20}$ ou alcényloxy en $C_2$—$C_{20}$, linéaire ou ramifié, un reste L-aminoacide, qui est lié par l'intermédiaire du groupe α-amino, ou un reste oligopeptide, avec jusqu'à 10 unités L-aminoacide qui est relié par l'intermédiaire du groupe α-amino de l'unité aminoacide terminale, et

$R^2$ représente un atome d'hydrogène, un groupe $H_2PO_3$ ou $HSO_3$, un groupe alkyle en $C_1$—$C_{20}$ ou alcényle en $C_2$—$C_{20}$ linéaire ou ramifié, qui est éventuellement substitué par un ou plusieurs restes phényle, un groupe aryle comportant 6 à 18 atomes de C, un groupe de formule générale (II):

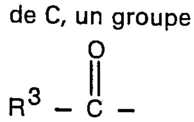

(II)

dans laquelle $R^3$ représente un groupe alkyle en $C_1$—$C_{20}$ ou alcényle en $C_2$—$C_{20}$ linéaire ou ramifié, qui est éventuellement substitué par un reste phényle, ou un groupe aryle en $C_6$—$C_{18}$, un reste nucléotide naturel comportant 1 à 3 groupes phosphate, qui est lié par l'intermédiaire du ou des groupes phosphate, ou un mono- ou disaccharide à liaison glycosidique;

à l'exception de la D-luciférine, de l'O-methylluciférine et de l'ester méthylique de la D-luciférine, caractérisé en ce que

(a) pour la préparation des composés de formule générale I, dans lesquels $R^2$ représente un atome d'hydrogène et $R^1$ représente un groupe alcoxy tel que défini ci-dessus, on fait réagir la luciférine avec un alcool approprié ($R^1$—H), en présence de dicyclohexylcarbodiimide dans le DMF,

(b) pour la préparation des composés de formule générale I, dans lesquels $R^2$ représente un atome d'hydrogène et $R^1$ représente un reste aminoacide tel que défini ci-dessus ou un oligopeptide, on fait réagir l'hydroxysuccinimidester de la D-luciférine avec l'aminoacide correspondant ou l'oligopeptide correspondant,

(c) pour la préparation des composés de formule générale I, dans lesquels $R^1$ représente un groupe hydroxyle et $R^2$ représente un groupe alkyle, alcényle ou aryle tel que défini ci-dessus, ou un groupe de formule générale II tel que défini ci-dessus, on fait réagir la luciférine avec le diazométhane, en un composé de formule générale I, où $R^1$ représente un groupe méthoxy,

on fait réagir ce dernier composé avec un chlorure d'alkyle, d'alcényle ou d'aryle ou avec un chlorure d'acide d'un composé répondant à la formule générale II définie ci-dessus, et on sépare le groupe méthyle de $R^1$ par voie enzymatique à l'aide de la carboxylestérase,

(d) pour la préparation des composés de formule générale I, dans lesquels $R^1$ représente un groupe hydroxyle et $R^2$ représente un reste mono- ou disaccharide, on fait réagir la luciférine, après protection du groupe carboxyle avec un $^1$Br-mono- ou disaccharide et ensuite, on sépare le groupe méthyle du reste $R^1$ à l'aide de la carboxylestérase,

(e) pour la préparation des composés de formule générale I, dans lesquels $R^1$ représente un groupe hydroxyle ou un groupe alcoxy ou alcènyle tel que défini ci-dessus et $R^2$ représente un nucléotide tel que

défini ci-dessus, on fait réagir un ester de luciférine, qui peut être l'ester méthylique de luciférine ou un ester correspondant au reste $R^1$, avec un nucléotide, selon le schéma suivant

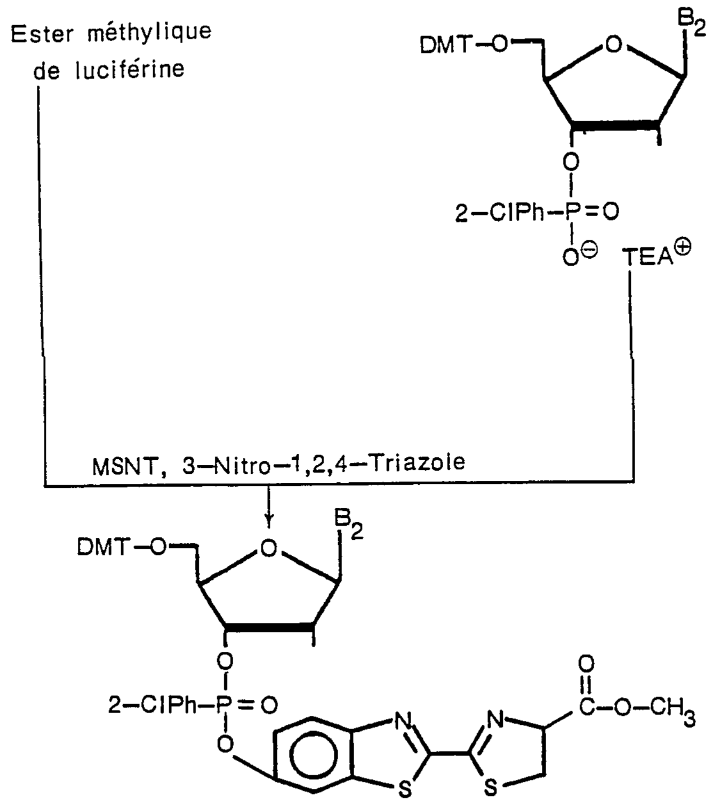


(où les symboles représentent:

$B_2$ = une base éventuellement protégée;

DMT = 4,4'-diméthoxytrityle;

TEA = triéthylammonium;

MSNT = 1(mésitylène-2-sulfonyl)-3-nitro-1,2,4-triazol

2-ClPh = 2-chlorophényle),

de manière connue en soi, on sépare les groupes de protection du composé obtenu et ensuite éventuellement, dans le cas de l'ester méthylique, on sépare le groupe méthyle à l'aide de l'estérase,

(f) pour la préparation des composés de formule générale I, dans lesquels $R^1$ représente un reste aminoacide tel que défini ci-dessus ou un oligopeptide tel que défini ci-dessus, et $R^2$ représente un nucléotide tel que défini ci-dessus, dans un dérivé de luciférine de formule générale I, dans lequel $R^1$ représente un reste aminoacide ou un oligopeptide, on protège les groupes fonctionnels de ce reste $R^1$ de manière connue en soi et ensuite, on effectue la réaction suivant le schéma représenté en (e),

(g) pour la préparation des composés de formule générale I, dans lesquels $R^1$ représente un groupe alcoxy ou alcényloxy tel que défini ci-dessus ou un reste aminoacide ou un oligopeptide et $R^2$ représente les groupes ou restes mentionnés ci-dessus, sauf un reste nucléotide, on introduit d'abord le reste $R^1$ comme mentionné ci-dessus et ensuite seulement, on introduit le reste $R^2$,

(h) pour la préparation des composés de formule générale I, dans lesquels $R^1$ représente un groupe hydroxyle et $R^2$ représente un groupe $HSO_3$, on fait réagir la luciférine avec un complexe pyridine-$SO_3$,

(i) pour la préparation des composés de formule générale I, dans lesquels $R^1$ représente un groupe hydroxyle et $R^2$ représente un groupe $H_2PO_3$, on fait réagir la luciférine avec l'oxychlorure de phosphore, et

(j) pour la préparation des composés de formule générale I, dans lesquels $R^1$ représente un groupe amino et $R^2$ représente un atome d'hydrogène, on fait réagir l'hydroxysuccinimidester de luciférine avec l'ammoniac.

2. Procédé selon la revendication 1, caractérisé en ce que l'on prépare des dérivés de luciférine de formule générale (I), dans laquelle

$R^1$ représente un groupe hydroxy ou amino, un groupe alcoxy en $C_1$—$C_6$ ou alcényloxy en $C_2$—$C_6$, un aminoacide naturel ou un oligopeptides d'aminoacides naturels, avec de préférence jusqu'à 5 unités aminoacide, et

$R^2$ représente un atome d'hydrogène, un groupe $H_2PO_3$ ou $HSO_3$, un groupe alkyle en $C_1$—$C_{20}$ ou

alcényle en $C_2$—$C_{20}$ linéaire ou ramifié, qui est éventuellement substitué par un ou plusieurs restes phényle, un groupe aryle avec 6 à 18 atomes de C, un groupe de formule générale (II):

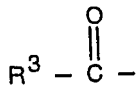 (II)

dans laquelle $R^3$ représente un groupe alkyle en $C_1$—$C_{20}$ ou alcényle en $C_2$—$C_{20}$ linéaire ou ramifié, qui est éventuellement substitué par un groupe phényle, ou un groupe aryle en $C_6$—$C_{18}$, ou un reste nucléotide naturel avec 1 à 3 groupes phosphate, qui est relié par l'intermédiaire du ou des groupes phosphate.

3. Procédé selon la revendication 1, caractérisé en ce que l'on prépare des dérivés de luciférine de formule générale (I), dans laquelle

$R^1$ représente un groupe hydroxy ou amino, un groupe alcoxy en $C_1$—$C_{20}$ ou alcényloxy en $C_2$—$C_{20}$, linéaire ou ramifié, un reste L-aminoacide qui est relié par l'intermédiaire du groupe α-amino, ou un reste oligopeptide avec jusqu'à 10 unités L'aminoacide, qui est relié par l'intermédiaire du groupe α-amino de l'unité aminoacide terminale, et

$R^2$ représente un atome d'hydrogène, un groupe $H_2PO_3$ ou $HSO_3$, un groupe alkyle en $C_1$—$C_6$ ou alcényle en $C_2$—$C_6$, linéaire ou ramifié, qui est éventuellement substitué par un reste phényle ou un groupe de formule générale (II):

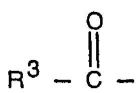 (II)

dans laquelle $R^3$ représente un groupe alkyle en $C_1$—$C_6$ ou alcényle en $C_2$—$C_6$, qui est éventuellement substitué par un reste phényle.

4. Procédé selon la revendication 1, caractérisé en ce que l'on prépare des dérivés de luciférine de formule générale (I), dans laquelle

$R^1$ représente un groupe hydroxy ou amino, un groupe alcoxy en $C_1$—$C_6$ ou alcényloxy en $C_2$—$C_6$, un reste aminoacide d'un aminoacide naturel ou un reste oligopeptide d'aminoacides naturels, avec de préférence jusqu'à 5 unités aminoacide, et

$R^2$ représente un atome d'hydrogène, un groupe $H_2PO_3$ ou $HSO_3$, un groupe alkyle en $C_1$—$C_6$ ou alcényle en $C_2$—$C_6$, linéaire ou ramifié, qui est éventuellement substitué par un reste phényle, ou un groupe de formule générale (II):

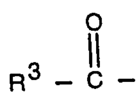 (II)

dans laquelle $R^3$ représente un groupe alkyle en $C_1$—$C_6$ ou alcényle en $C_2$—$C_6$, qui est éventuellement substitué par un reste phényle.

5. Procédé selon la revendication 1, caractérisé en ce que l'on prépare des dérivés de luciférine de formule générale (I), dans laquelle

$R^1$ représente un groupe hydroxy ou amino, un groupe alcoxy en $C_1$—$C_6$ ou un groupe de formule générale (III)

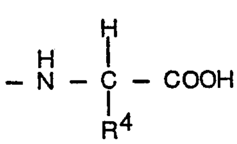 (III)

dans laquelle

$R^4$ représente

$-H$, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2\ C_6H_5$, 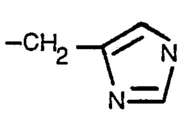 , 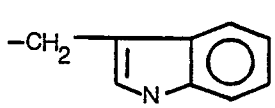 , $-CH_2SH$,

$-CH_2CH_2CH_2-NH-C(=NH)-NH_2$ ou $-CH_2CH_2SCH_3$,

ou dans laquelle le groupe de formule générale (III) est un reste proline ou hydroxyproline, et

$R^2$ représente un atome d'hydrogène, un groupe alkyle en $C_1$—$C_6$ qui est éventuellement substitué par un reste phényle, un groupe phényle ou naphtyle, un groupe $H_2PO_3$ ou $HSO_3$ ou un groupe de formule générale (II):

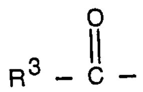

(II)

dans laquelle $R^3$ représente un groupe alkyle en $C_1$—$C_6$ éventuellement substitué par un reste phényle.

6. Procédé selon la revendication 1, caractérisé en ce que l'on prépare des dérivés de luciférine de formule générale (I), dans laquelle $R^2$ représente un atome d'hydrogène, dans le cas où $R^1$ représente un autre reste que le groupe hydroxy, ou dans laquelle $R^1$ représente un groupe hydroxy, dans le cas où $R^2$ représente un autre reste qu'un atome d'hydrogène.

7. Procédé selon la revendication 1, caractérisé en ce que l'on prépare les dérivés de luciférine de formule générale (I) suivants:

la D-luciféryl-L-$N^{\alpha}$-arginine,
la D-luciféryl-L-phénylalanine,
la D-luciféryl-L-méthionine,
la D-luciféryl-L-glycine,
le O-sulfate de D-luciférine,
le O-phosphate de D-luciférine.

8. Utilisation des dérivés de luciférine de formule générale (I) selon l'une quelconque des revendications 1 à 7, et de la O-méthylluciférine ainsi que de l'éther méthylique de D-luciférine, pour la détermination de substances ayant une activité biochimique, pour la détection de ligands, avec lesquels est associée une enzyme, avec formation d'un composé conjugué ligand-enzyme, qui est capable de libérer la luciférine à partir des dérivés de luciférine ou de l'ester méthylique de la D-luciférine ainsi que de la O-méthylluciférine mentionnée.

9. Utilisation selon la revendication 8, dans des dosages immunologiques pour la détection de composés conjugués anticorps-, antigène- ou haptène-enzyme, dans des procédés d'absorption ou dans des procédés d'hybridation d'acides nucléiques.

10. Procédé de détection de ligands pour la détermination de substances ayant une activité biochimique, dans lequel on marque le ligand au moyen d'une enzyme, avec formation d'un composé conjugué ligand-enzyme, caractérisé en ce que l'on met en oeuvre une enzyme qui peut libérer la luciférine à partir des dérivés de luciférine de formule générale (I), selon l'une quelconque des revendications 1 à 7, à partir de la O-méthylluciférine ou de l'ester méthylique de la D-luciférine,

à l'aide d'un composé conjugué d'enzyme, on libère la luciférine à partir d'un dérivé de luciférine ou à partir de l'ester méthylique de D-luciférine ou à partir de la O-méthylluciférine,

on fait réagir la luciférine libérée avec l'enzyme luciférase du ver luisant photinus pyralis,

on évalue quantitativement la quantité de lumière émise ici par rayonnement, en particulier par un moyen luminométrique, et

à l'aide des données obtenues, on détermine la substance à déterminer.

11. Procédé selon la revendication 10, caractérisé en ce que comme enzyme libérant la luciférine, on utilise l'estérase, la carboxypeptidase A, la carboxypeptidase B, l'arylsulfatase, les phosphatases alcalines et acides, les lipases, l'acétylestérase, les nucléotidases, la kininase II, les α- et β-glucosidases, les α/β-amylases, les amidases, l'aminoacylase I, la phospholipase A—D; les nucléases et/ou les protéinases microbiennes.

12. Procédé selon la revendication 10, pour la détection d'antigènes, d'haptènes ou d'anticorps, qui sont marqués par une enzyme avec formation d'un produit conjugué d'enzyme, dans des dosages immunologiques, caractérisé en ce que, à l'aide du produit conjugué d'enzyme, on libère la luciférine à partir d'un dérivé de luciférine ou à partir de l'ester méthylique de la D-luciférine ou à partir de la O-méthylluciférine,

on fait réagir la luciférine libérée avec l'enzyme luciférase du ver luisant photinus pyralis,

on évalue quantitativement la quantité de lumière émise ici par rayonnement, en particulier par un moyen luminométrique, et

à l'aide des données obtenues, on évalue la quantité de l'antigène, de l'anticorps ou de l'haptène à déterminer.

**Claims for the Contracting States: BE CH DE FR GB IT LI LU NL SE**

1. D-luciferin derivatives of the General Formula (I):

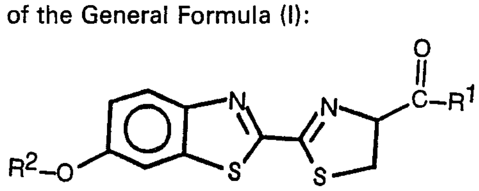

(I)

wherein

$R^1$ signifies a hydroxy or amino group, a linear or branched $C_1$—$C_{20}$ alkoxy or $C_2$—$C_{20}$ alkenyloxy group, an L-amino acid radical bound by the α-amino group, or an oligopeptide radical with up to 10 L-amino acid units, bound by the α-amino group of the terminal amino acid unit and

$R^2$ is a hdyrogen atom, a $H_2PO_3$ or $HSO_3$ group, a linear or branched $C_1$—$C_{20}$ alkyl or $C_2$—$C_{20}$ alkenyl group, optionally substituted by one or several phenyl radicals, an aryl group with 6 to 18 C atoms, a group of the General Formula (II):

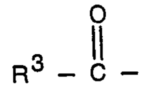

(II)

wherein $R^3$ is a linear or branched $C_1$—$C_{20}$ alkyl or a $C_2$—$C_{20}$ alkenyl group optionally substituted by a phenyl radical, or a $C_6$—$C_{18}$ aryl group;

a naturally occurring nucleotide radical with 1 to 3 phosphate groups attached by means of the phosphate group or groups, or

a glycosidically attached mono- or disaccharide; with the exception of D-luciferin, O-Methylluciferin and D-luciferinmethylester.

2. Luciferin derivatives according to Claim 1 of General Formula (I), wherein

$R^1$ represents a hydroxy or amino group, a $C_1$—$C_6$ alkoxy or $C_2$—$C_6$ alkenyloxy group, a naturally occurring amino acid or an oligopeptide of naturally occurring amino acids preferably with up to 5 amino acid units and

$R^2$ is a hydrogen atom, a $H_2PO_3$ or $HSO_3$ group, a linear or branched $C_1$—$C_{20}$ alkyl or $C_2$—$C_{20}$ alkenyl group, optionally substituted with one or several phenyl radicals, an aryl group with 6 to 18 C atoms, a group of the General Formula (II):

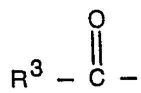

(II)

wherein $R^3$ is a linear or branched $C_1$—$C_{20}$ alkyl or $C_2$—$C_{20}$ alkenyl group, optionally substituted by a phenyl radical, or a $C_6$—$C_{18}$ aryl group;

or a naturally occurring nucleotide radical with 1 to 3 phosphate groups attached by means of the phosphate group or groups.

3. Luciferin derivatives according to Claim 1 of General Formula (I), wherein

$R^1$ represents a hydroxy or amino group, a linear or branched $C_1$—$C_{20}$ alkoxy or a $C_2$—$C_{20}$ alkenyloxy group, an L-amino acid radical attached by means of the α-amino group, or an oligopeptide radical with up to 10 L-amino acid units, attached by means of the α-amino group of the terminal amino acid unit, and

$R^2$ is a hydrogen atom, a $H_2PO_3$ or $HSO_3$ group, a linear or branched $C_1$—$C_6$ alkyl or a $C_2$—$C_6$ alkenyl group, optionally substituted by a phenyl radical, or a group of General Formula (II):

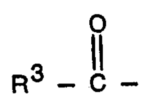

(II)

wherein $R^3$ signifies a $C_1$—$C_6$ alkyl or a $C_2$—$C_6$ alkenyl group, optionally substituted by a phenyl radical.

4. Luciferin derivatives according to Claim 1 of General Formula (I), wherein

$R^1$ is a hydroxy or amino group, a $C_1$—$C_6$ alkoxy or a $C_2$—$C_6$ alkenyloxy group, an amino acid radical of a naturally occurring amino acid or an oligopeptide radical of naturally occurring amino acids with preferably up to 5 amino acid groups and

$R^2$ is a hydrogen atom, a $H_2PO_3$ or $HSO_3$ group, a linear or branched $C_1$—$C_6$ alkyl or $C_2$—$C_6$ alkenyl group, optionally substituted by a phenyl radical, or a group of General Formula (II):

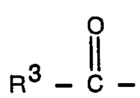

(II)

wherein $R^3$ is a $C_1$—$C_6$ alkyl or a $C_2$—$C_6$ alkenyl group, optionally substituted by a phenyl radical.

5. Luciferin derivatives according to Claim 1 of General Formula (I), wherein

$R^1$ represents a hydroxy or an amino group, a $C_1$—$C_6$ alkoxy group or a group of General Formula (III)

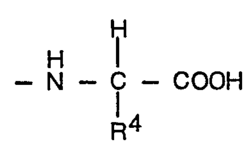

(III)

wherein $R^4$ stands for

$-H$, $-CH_3$, $-CH_2OH$, $-CH(OH)CH_3$, $-CH(CH_3)_2$, $-CH_2CH(CH_3)_2$, $-CH(CH_3, -CH_2CH_3)$, $-CH_2COOH$, $-CH_2CONH_2$, $-CH_2CH_2COOH$, $-CH_2CH_2CONH_2$, $-CH_2CH_2CH_2CH_2NH_2$, $-CH_2C_6H_4OH$,

$-CH_2\ C_6H_5$, 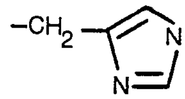 , 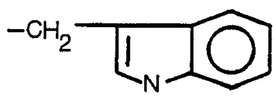 , $-CH_2SH$,

$-CH_2CH_2CH_2-NH-C(=NH)-NH_2$ or $-CH_2CH_2SCH_3$,

or wherein the group of General Formula (III) is a proline or hydroxyproline radical, and

$R^2$ is a hydrogen atom, a $C_1$—$C_6$ alkyl group optionally substituted by a phenyl residue, a phenyl or naphthyl group, a $H_2PO_3$, or $HSO_3$ group or a group of Formula (II)

$$R^3 - \overset{\overset{\displaystyle O}{\|}}{C} - \qquad (II)$$

wherein $R^3$ is a $C_1$—$C_6$ alkyl group optionally substituted by a phenyl radical.

6. Luciferin derivatives according to Claim 1 of General Formula (I) wherein $R^2$ signifies a hydrogen atom, if $R^1$ represents a radical other than a hydroxy group, or wherein $R^1$ is a hydroxy group, if $R^2$ represents a radical other than a hydrogen atom.

7. Luciferin derivatives according to Claim 1 of General Formula (I), i.e.
D-luciferyl-L-$N^{\alpha}$-arginine
D-luciferyl-L-phenylalanine
D-luciferyl-L-methionine
D-luciferyl-L-glycine
D-luciferin-O-sulfate
D-luciferin-O-phosphate.

8. Use of the luciferin derivatives of General Formula (I) according to one of Claims 1 to 7 and of O-methyl luciferin as well as of D-luciferinmethylester to determine biochemically active substances for the detection of ligands to which an enzyme is attached while forming a ligand-enzyme conjugate, said enzyme being capable of releasing luciferin from said luciferin derivatives, said luciferinmethylester or said O-methyl luciferin.

9. Use according to Claim 8 in immuno assays for the detection of antibody, antigen or hapten-enzyme conjugates, in blot methods or in methods for hybridizing nucleic acids.

10. Process for the detection of ligands in the determination of biochemically active substances, wherein the ligand is marked by an enzyme while forming a ligand-enzyme conjugate, characterized in that an enzyme is used that is capable of releasing luciferin from the luciferin derivatives of General Formula (I) according to one of Claims 1 to 7, from D-luciferinmethylester or from O-methylluciferin,

with the aid of the enzyme conjugate, luciferin is released from a luciferin derivative, from D-luciferinmethylester, or from O-methylluciferin,

the luciferin released is reacted with the enzyme luciferase of the fire fly Photinus pyralis,

the amount of light emitted is measured quantitatively, in particular luminometrically, and

the quantity of the substance to be detected is determined from the data obtained.

11. Process according to Claim 10, characterized in that as the enzyme releasing luciferin, esterases, carboxypeptidase A, carboxypeptidase B, arylsulfatase, alkaline and acid phosphatases, lipases, acetylesterase, nucleotidases, kininase II, α- and β-glucosidases, α/β-amylases, amidases, aminoacylase I, phospholipase A—D, nucleases and/or microbial proteinases are used.

12. Process, according to Claim 10 for the detection of antigens, haptens or antibodies marked with an enzyme while forming an enzyme conjugate in immuno assays, characterized in that luciferin is released by means of the enzyme conjugate from a luciferin derivative, from D-luciferinmethylester, or from O-methylluciferin,

the luciferin released is reacted with the enzyme luciferase of the fire fly Photinus pyralis,

the amount of light emitted is determined quantitatively, in particular luminometrically, and

the quantity of the antigen, antibody or hapten to be detected is determined from the data obtained.

**Claims for the Contracting State: AT**

1. Process for producing D-luciferin derivatives of the following General Formula (I):

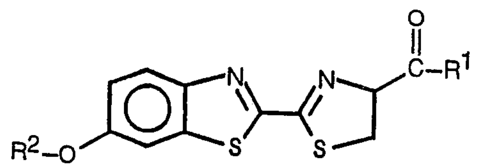

(I)

wherein

$R^1$ signifies a hydroxy or amino group, a linear or branched $C_1$—$C_{20}$ alkoxy or $C_2$—$C_{20}$ alkenyloxy group, an L-amino acid radical bound by the α-amino group, or an oligopeptide radical with up to 10 L-amino acid units, bound by the α-amino group of the terminal amino acid unit and

$R^2$ is a hydrogen atom, a $H_2PO_3$ or $HSO_3$ group, a linear or branched $C_1$—$C_{20}$ alkyl or $C_2$—$C_{20}$ alkenyl group, optionally substituted by one or several phenyl radicals, an aryl group with 6 to 18 C atoms, a group of the General Formula (II):

$$R^3 - \overset{O}{\overset{\|}{C}} - \qquad (II)$$

wherein $R^3$ is a linear or branched $C_1$—$C_{20}$ alkyl or a $C_2$—$C_{20}$ alkenyl group optionally substituted by a phenyl radical, or a $C_6$—$C_{18}$ aryl group;

a naturally occurring nucleotide radical with 1 to 3 phosphate groups attached by means of the phosphate group or groups, or

a glycosidically attached mono- or disaccharide; with the exception of D-luciferin, O-methylluciferin and D-luciferinmethylester; characterized in that

(a) for producing the compounds of the General Formula I, wherein $R^2$ is a hydrogen atom and $R^1$ represents an alkoxy group as defined above, one reacts luciferin with the corresponding alcohol ($R^1$—H) in the presence of dicyclohexylcarbodiimide,

(b) for producing the compounds of the General Formula I, wherein $R^2$ is a hydrogen atom and $R^1$ represents an amino acid radical or an oligopeptide as defined above, one reacts D-luciferin hydroxysuccinimid ester with the corresponding amino acid or the corresponding oligopeptide,

(c) for producing the compounds of the General Formula I, wherein $R^1$ is a hydroxy group and $R^2$ is an alkyl, alkenyl or aryl group as defined above or a group of the General Formula II as defined above, one reacts luciferin with diazomethane to give a compound of the General Formula I, wherein $R^1$ is a methoxy group, one reacts the latter compound with an alkyl, alkenyl or arylchloride or an acid chloride of a compound of the above given General Formula II and cleaves off the methyl group of $R^1$ enzymatically with carboxyl esterase,

(d) for producing the compounds of the General Formula I, wherein $R^1$ is a hydroxy group and $R^2$ is a mono or disaccharide radical, one reacts luciferin the carboxyl group of which has been protected with a $^1$Br-mono or disaccharide and then cleaves off the methyl group of the radical $R^1$ with carboxyl esterase,

(e) for producing the compounds of the General Formula I, wherein $R^1$ is a hydroxy group or an alkoxy or alkenyloxy group as defined above and $R^2$ is a nucleotide radical as defined above, one reacts a luciferin

ester, which may be the luciferin methyl ester or an ester corresponding to the radical $R^1$, with a nucleotide according to the following scheme

SCHEMA

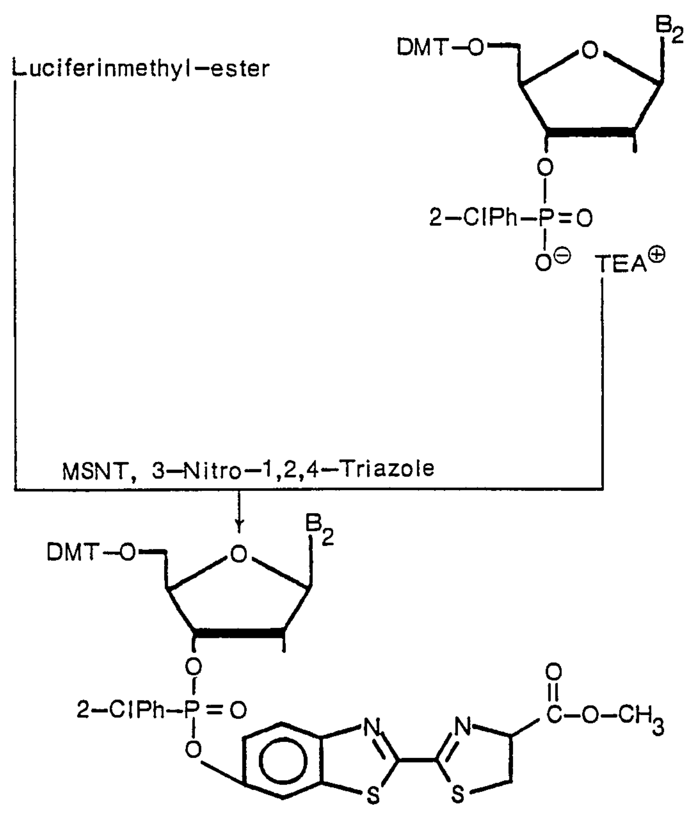


(In the above scheme
$B_2$ = optionally protected base;
DMT = 4,4'-dimethoxytrityl;
TEA = triethylammonium;
MSNT = 1(mesitylen-2-sulfonyl)-3-nitro-1,2,4-triazole
2-ClPh = 2-chlorophenyl)
cleaves off the protective groups from the thus obtained compound in a manner known per se and optionally cleaves off the methyl group with esterase in case of the methyl ester,

(f) for producing the compounds of the General Formula I, wherein $R^1$ represents an amino acid radical defined above or an oligopeptide defined above and $R^2$ is a nucleotide as defined above, one protects, in a manner known per se, in a luciferin derivative of the General Formula I, wherein $R^1$ is an amino acid radical or an oligopeptide, the functional groups of said radical $R^1$ and then reacts according to the scheme shown in (e),

(g) for producing the compounds of the General Formula I, wherein $R^1$ represents an alkoxy or alkenyloxy radical or an amino acid radical or an oligopeptide as defined above and $R^2$ stands for the above given groups or radicals, however not for a nucleotide radical, one introduces first the radical $R^1$ as described above and then the radical $R^2$,

(h) for producing the compound of the General Formula I, wherein $R^1$ is a hydroxy group and $R^2$ is a $HSO_3$ group, one reacts luciferin with a pyridin-$SO_3$-complex,

(i) for producing the compound of the General Formula I, wherein $R^1$ is a hydroxy group and $R^2$ is a $H_2PO_3$ group, one reacts luciferin with phosporoxychloride, and

(j) for producing the compound of the General Formula I, wherein $R^1$ is an amino group and $R^2$ is a hydrogen atom, one reacts luciferin hydroxy succinimid ester with ammonia.

2. Process according to Claim 1, characterized in that one produces luciferin derivatives of the General Formula I, wherein

$R^1$ represents a hydroxy or amino group, a $C_1$—$C_6$ alkoxy or $C_2$—$C_6$ alkenyloxy group, a naturally occurring amino acid or an oligopeptide of naturally occurring amino acids preferably with up to 5 amino acid units and

$R^2$ is a hydrogen atom, a $H_2PO_3$ or $HSO_3$ group, a linear or branched $C_1$—$C_{20}$ alkyl or $C_2$—$C_{20}$ alkenyl

group, optionally substituted with one or several phenyl radicals, an aryl group with 6 to 18 C atoms, a group of the General Formula (II):

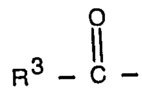

(II)

wherein $R^3$ is a linear or branched $C_1$—$C_{20}$ alkyl or $C_2$—$C_{20}$ alkenyl group, optionally substituted by a phenyl radical, or a $C_6$—$C_{18}$ aryl group;
or a naturally occurring nucleotide radical with 1 to 3 phosphate groups attached by means of the phosphate group or groups.

3. A process according to Claim 1, characterized in that one produces luciferin derivatives of the General Formula I, wherein

$R^1$ represents a hydroxy or amino group, a linear or branched $C_1$—$C_{20}$ alkoxy or a $C_2$—$C_{20}$ alkenyloxy group, an L-amino acid radical attached by means of the α-amino group, or an oligopeptide radical with up to 10 L-amino acid units, attached by means of the α-amino group of the terminal amino acid unit, and

$R^2$ is a hydrogen atom, a $H_2PO_3$ or $HSO_3$ group, a linear or branched $C_1$—$C_6$ alkyl or a $C_2$—$C_6$ alkenyl group, optionally substituted by a phenyl radical, or a group of General Formula (II):

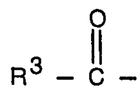

(II)

wherein $R^3$ signifies a $C_1$—$C_6$ alkyl or a $C_2$—$C_6$ alkenyl group, optionally substituted by a phenyl radical.

4. A process according to Claim 1, characterized in that one produces luciferin derivatives of the General Formula I, wherein

$R^1$ is a hydroxy or amino group, a $C_1$—$C_6$ alkoxy or a $C_2$—$C_6$ alkenyloxy group, an amino acid radical of a naturally occurring amino acid or an oligopeptide radical of naturally occurring amino acids with preferably up to 5 amino acid groups and

$R^2$ is a hydrogen atom, a $H_2PO_3$ or $HSO_3$ group, a linear or branched $C_1$—$C_6$ alkyl or a $C_2$—$C_6$ alkenyl group, optionally substituted by a phenyl radical, or a group of General Formula (II):

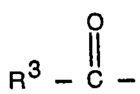

(II)

wherein $R^3$ signifies a $C_1$—$C_6$ alkyl or a $C_2$—$C_6$ alkenyl group, optionally substituted by a phenyl radical.

5. A process of Claim 1, characterized in that one produces luciferin derivatives of the General Formula I, wherein

$R^1$ represents a hydroxy or an amino group, a $C_1$—$C_6$ alkoxy group or a group of General Formula (III)

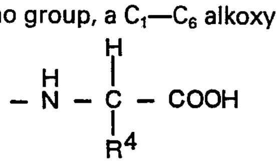

(III)

wherein $R^4$ stands for —H, —$CH_3$, $CH_2OH$, $CH(OH)CH_3$, —$CH(CH_3)_2$, —$CH_2CH(CH_3)_2$, —$CH(CH_3,CH_2CH_3)$, —$CH_2COOH$, —$CH_2CONH_2$, —$CH_2CH_2COOH$, $CH_2CH_2CONH_2$, —$CH_2CH_2CH_2CH_2NH_2$, —$CH_2C_6H_4OH$, —$CH_2C_6H_5$,

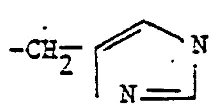 , 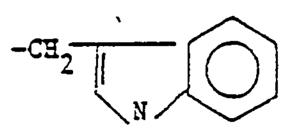 , $-CH_2SH$,

—$CH_2CH_2CH_2NH$—$C(=NH)$—$NH_2$ or —$CH_2CH_2SCH_3$,
or wherein the group of General Formula (III) is a proline or hydroxyproline radical, and

$R^2$ is a hydrogen atom, a $C_1$—$C_6$ alkyl group optionally substituted by a phenyl radical, a phenyl or naphthyl group, a $H_2PO_3$ or $HSO_3$ group or a group of the Formula (II)

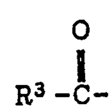

(II)

wherein $R^3$ is a $C_1$—$C_6$ alkyl group optionally substituted by a phenyl radical.

6. A process according to Claim 1, characterized in that one produces luciferin derivatives of the General Formula I, wherein $R^2$ signifies a hydrogen atom, if $R^1$ represents a radical other than a hydroxy group, or wherein $R^1$ is a hydroxy group, if $R^2$ represents a radical other than a hydrogen atom.

7. A process according to Claim 1, characterized in that one produces the following luciferin derivatives of the General Formula I:
D-luciferyl-L-Nα-arginine
D-luciferyl-L-phenylalanine
D-luciferyl-L-methionine
D-luciferyl-L-glycine
D-luciferin-O-sulfate
D-luciferin-O-phosphate.

8. Use of the luciferin derivatives of General Formula I according to one of Claims 1 to 7 and of O-methyl luciferin as well as of D-luciferinmethylester to determine biochemically active substances for the detection of ligands to which an enzyme is attached while forming a ligand-enzyme conjugate, said enzyme being capable of releasing luciferin from said luciferin derivatives, said luciferinmethylester or said O-methyl luciferin.

9. Use according to Claim 8 in immuno assays for the detection of antibody, antigen or hapten-enzyme conjugates, in blot methods or in methods for hybridizing nucleic acids.

10. Process for the detection of ligands in the determination of biochemically active substances, wherein the ligand is marked by an enzyme while forming a ligand-enzyme conjugate, characterized in that an enzyme is used that is capable of releasing luciferin from the luciferin derivatives of General Formula I according to one of Claims 1 to 7, from D-luciferinmethylester or from O-methylluciferin,

with the aid of the enzyme conjugate, luciferin is released from a luciferin derivative, from D-luciferinmethylester, or from O-methylluciferin,

the luciferin released is reacted with the enzyme luciferase of the fire fly Photinus pyralis,

the amount of light emitted is measured quantitatively, in particular luminometrically, and

the quantity of the substance to be detected is determined from the data obtained.

11. Process according to Claim 10, characterized in that as the enzyme releasing luciferin, esterases, carboxypeptidase A, carboxypeptidase B, arylsulfatase, alkaline and acid phosphatases, lipases, acetylesterase, nucleotidases, kininase II, α- and β-glucosidases, α/β-amylases, amidases, aminoacylase I, phospholipase A—D, nucleases and/or microbial proteinases are used.

12. Process according to Claim 10 for the detection of antigens, haptens or antibodies marked with an enzyme while forming an enzyme conjugate in immuno assays, characterized in that luciferin is released by means of the enzyme conjugate from a luciferin derivative, from D-luciferinmethylester, or from O-methylluciferin, the luciferin released is reacted with the enzyme luciferase of the fire fly Photinus pyralis, the amount of light emitted is determined quantitatively, in particular luminometrically, and the quantity of the antigen, antibody or hapten to be detected is determined from the data obtained.

**Figur 1** - Beispiel A

Kurve für die Bindung von Bradykinin im kompetitiven Fest-phasen-Biolumineszenz-Enzym-Immunoassay (competitive solide phase bioluminescence enzyme immunoassay; BEIA)

* RIA = Radioimmunoassay; derzeitige Nachweisgrenze

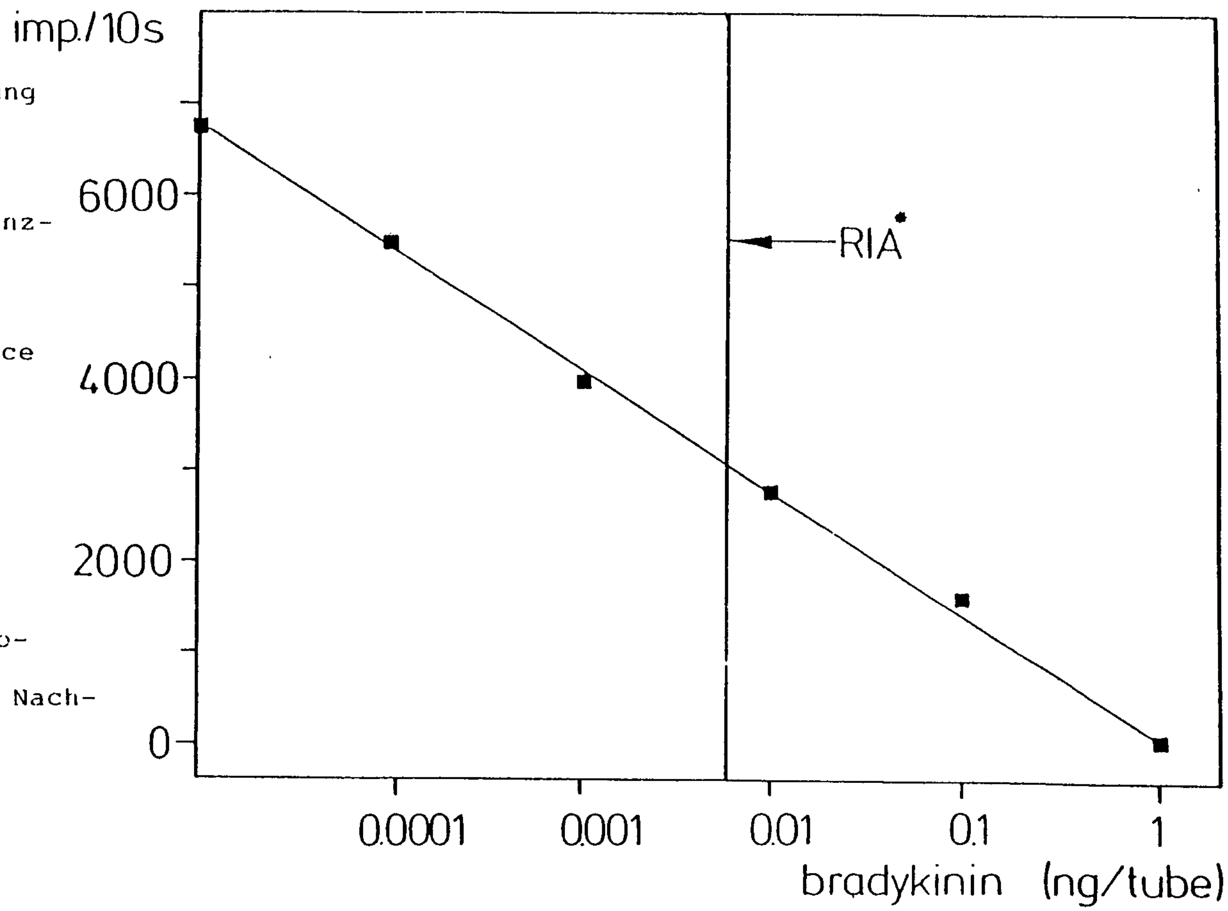


* Lit.: K. Shimamoto et al. (1978) J. Lab. Clin. Med. 91, 721 728

Figur 2 - Beispiel B

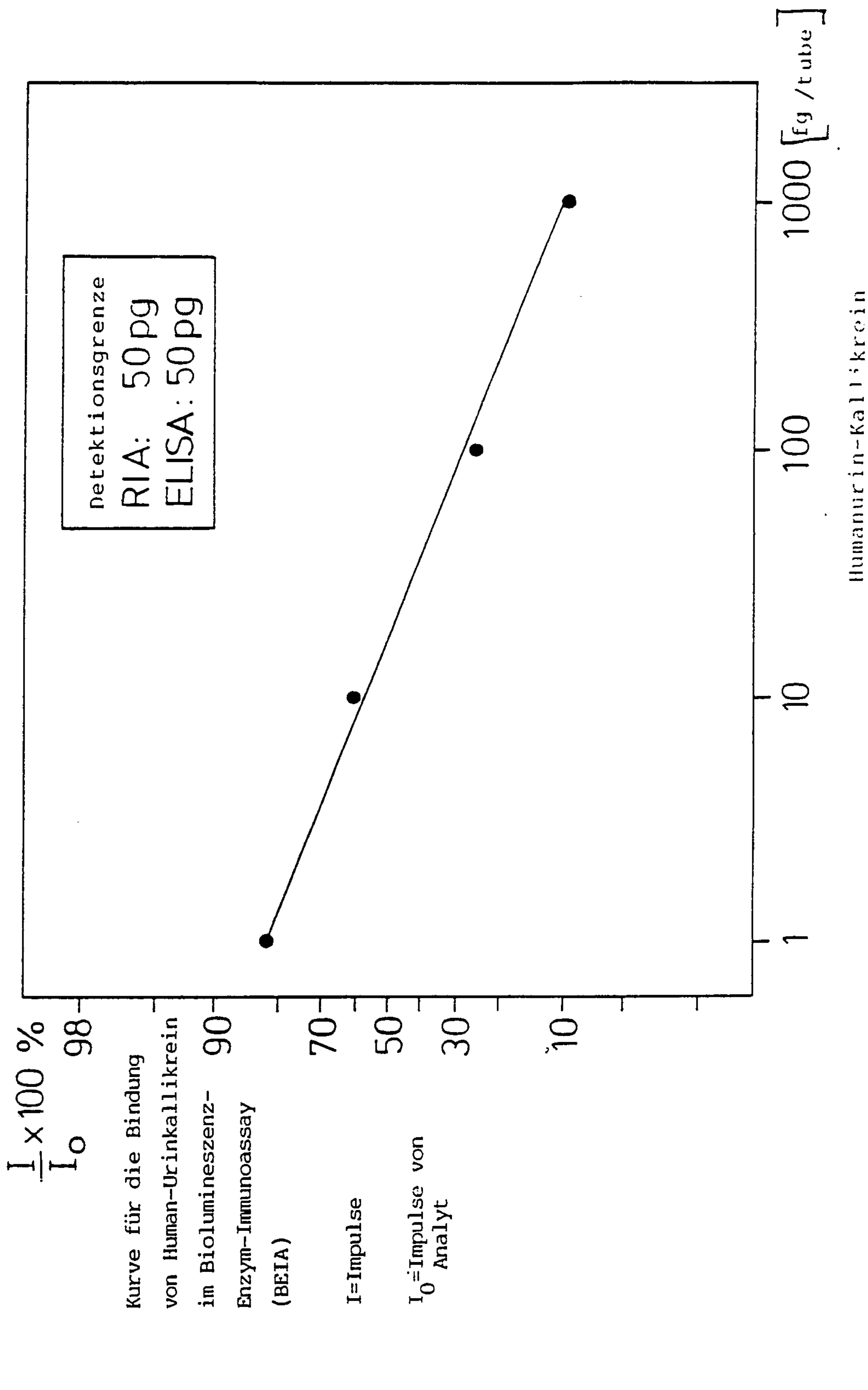

**Figur 3 - Beispiel C**

Titerbestimmung von HUK-Antiserum mittels BEIA